(19) Europäisches Patentamt
European Patent Office
Office européen des brevets

(11) EP 1 130 096 A1

(12) EUROPEAN PATENT APPLICATION

(43) Date of publication:
**05.09.2001 Bulletin 2001/36**

(21) Application number: **00400588.0**

(22) Date of filing: **03.03.2000**

(51) Int Cl.$^7$: **C12N 15/12**, C07K 14/47, C07K 16/18, C12Q 1/68, C12N 15/11, C12N 5/10, C12N 15/85, A61K 38/17, A61P 35/00

(84) Designated Contracting States:
**AT BE CH CY DE DK ES FI FR GB GR IE IT LI LU MC NL PT SE**
Designated Extension States:
**AL LT LV MK RO SI**

(71) Applicant: **Institut de la Santé et de la Recherche Médicale**
**75654 Paris Cedex 13 (FR)**

(72) Inventor: **Crisanti-Lassiaz, Patricia**
**75015 Paris (FR)**

(74) Representative: **Peaucelle, Chantal et al**
**Cabinet Armengaud Aine**
**3, avenue Bugeaud**
**75116 Paris (FR)**

(54) **Proliferation arrest trancription factor (PATF) and its use**

(57) The present invention relates to new compounds useful for controlling cell proliferation and/or differentiation. It namely relates to polypeptides which:

- comprise at least one sequence of the leucine zipper domain type at the N-terminus,
- comprise at least one sequence of the basic domain type at the C-terminus, and which
- comprise at least one sequence of the nuclearization signal type and/or are coupled to a compound capable of performing a nuclearization of said polypeptide.

EP 1 130 096 A1

SEQUENCE LISTING

<110> I.N.S.E.R.M.

<120> Nouveau facteur de transcription PATF

<130> 1087EP99

<140>
<141>

<160> 18

<170> PatentIn Ver. 2.1

<210> 1
<211> 2196
<212> DNA
<213> Quail coturnix coturnix japonica

<220>
<221> CDS
<222> (752)..(1585)
<223> Proliferation Arrest Transcription Factor

<220>
<221> misc_feature
<222> (429)..(1082)
<223> fragment inserted in the RCAS vector for antisense strategy

<220>
<221> misc_feature
<222> (766)..(829)
<223> Leucine Zipper Domain

<220>
<221> misc_feature
<222> (1429)..(1495)
<223> Basic Domain

<220>
<221> misc_feature
<222> (1438)..(1453)
<223> Nuclear Localization Signal

<400> 1
cagcagcagt acttgaatgg cttagggcag agagtctgaa gagagtcgag ttgcacagtg 60

aaaatatata tttttatacc tagcacaact tccttgtata tttttggggt tcttttattg 120

tttgtttgtt ttttttcctt ttattttttc tttttccttt cctttttttgt tttggttctt 180

cttcgatctg ggttcgtttt attacttctt cttattacta ggaaggcaaa cttctaaaat 240

gtgaagggcg ttgggtttct ccttatctgg tagatgaggg caagcatttt gcactaatgt 300

ttcctgtgtg tggaggattc atggtatgtt tggaacatgt tacgtggata atgattgtat 360

tcttccgaca aacacaacca agttccccct ttggaaatac tgaatgtaaa tgagaacaga 420

```
gcagtccatc gatttgtatg cggctttcct gagtgcctta gaatctcttg atctcttttg 480

ttccttcttt tgttgtcggt ttggtttggt ttgggttttt gttgggtttt ctttttctt 540

ttccaaggaa aacagatgga gagaatcagc tcataaccac ctccttcctc ctctttgttt 600

acgatactat tttaaattat agaattcggg gctgctttat aatgtgaata ggaaactgcg 660

ttcatctagg tctgatagtg taaaatcaga gcatcagtat ttatggtatg tctgcacctt 720

tattcctatg cttgactatt agcaatatta c atg tat att ata tat cta aag    772
                                   Met Tyr Ile Ile Tyr Leu Lys
                                     1               5

tta tac caa gca cct tta aag aaa tca gag cgc gtg ctc cgt gca gtc   820
Leu Tyr Gln Ala Pro Leu Lys Lys Ser Glu Arg Val Leu Arg Ala Val
        10                  15                  20

ccc aca tcc cta cat ctc cat atc cat gca tcc cta tat cct gac atc   868
Pro Thr Ser Leu His Leu His Ile His Ala Ser Leu Tyr Pro Asp Ile
    25                  30                  35

cct aca tca ccg cat ccc tat atc act gta tct cca cat ccc tgt atc   916
Pro Thr Ser Pro His Pro Tyr Ile Thr Val Ser Pro His Pro Cys Ile
 40                  45                  50                  55

ctc ata tcc cca cat atc tcc tta tcc cca cac ccc cat ttc cca gct   964
Leu Ile Ser Pro His Ile Ser Leu Ser Pro His Pro His Phe Pro Ala
                 60                  65                  70

ccc agc agc tgt agg acc ctc ctc ctc cca gct gtg tcc cgg cag cag   1012
Pro Ser Ser Cys Arg Thr Leu Leu Leu Pro Ala Val Ser Arg Gln Gln
            75                  80                  85

cgg gtg ccg tcc cca tcc cac tat ggg gtg cac atg gac tgt ggc ctc   1060
Arg Val Pro Ser Pro Ser His Tyr Gly Val His Met Asp Cys Gly Leu
        90                  95                  100

atg gag cca ccg agc agg gaa tcg att gcc ttt cct gat cgt ttt cct   1108
Met Glu Pro Pro Ser Arg Glu Ser Ile Ala Phe Pro Asp Arg Phe Pro
    105                 110                 115

gct aag ctt atg ttg gga ttg ctt ata ttt aca gaa ata act tgc tgt   1156
Ala Lys Leu Met Leu Gly Leu Leu Ile Phe Thr Glu Ile Thr Cys Cys
120                 125                 130                 135

gag cgg aga agg gtg tat ggc cca tta acc aac cca aaa ccc cag agc   1204
Glu Arg Arg Arg Val Tyr Gly Pro Leu Thr Asn Pro Lys Pro Gln Ser
                140                 145                 150

ctc tcg gct ata aat cct aaa ggc ggc aca gag aac tca cgt ctc ttc   1252
Leu Ser Ala Ile Asn Pro Lys Gly Gly Thr Glu Asn Ser Arg Leu Phe
            155                 160                 165

tgg ggc tcc aac tgc tgt tta gaa gtg ttc ctg tcg tat aga tgt gtc   1300
Trp Gly Ser Asn Cys Cys Leu Glu Val Phe Leu Ser Tyr Arg Cys Val
        170                 175                 180

acg ttg tca gcc cag ggt cgg ggc acc cca aag cca ggg ctg tca gga   1348
Thr Leu Ser Ala Gln Gly Arg Gly Thr Pro Lys Pro Gly Leu Ser Gly
    185                 190                 195
```

```
gct ccg tgc acc gat agg cgt gcc atg agt ttc ttc cac tgt tat aga   1396
Ala Pro Cys Thr Asp Arg Arg Ala Met Ser Phe Phe His Cys Tyr Arg
200                 205                 210                 215

agc att ttg cac acc aag gtt ggt ttt tat gtc aaa att aag aag aaa   1444
Ser Ile Leu His Thr Lys Val Gly Phe Tyr Val Lys Ile Lys Lys Lys
                220                 225                 230

aaa aga aaa aaa aaa aga aat aat aat aat aat aat aaa aat aat aat   1492
Lys Arg Lys Lys Lys Arg Asn Asn Asn Asn Asn Asn Lys Asn Asn Asn
            235                 240                 245

aat aaa tat ata tat ata tta aaa aga aaa gaa aac aaa gcc aaa act   1540
Asn Lys Tyr Ile Tyr Ile Leu Lys Arg Lys Glu Asn Lys Ala Lys Thr
        250                 255                 260

tgc aat act gtt ttt agc aga cgg aat aaa tcc acg aac aaa taa       1585
Cys Asn Thr Val Phe Ser Arg Arg Asn Lys Ser Thr Asn Lys
    265                 270                 275

cgagatgaaa ggaatgaatg agtgggtgga aaacgacgcc tgtagggaaa ggctctgaac 1645

tgtcctttgg ggcagggggc gttgggttgg gtcagagtgc tgaccaactt gggctgcttc 1705

aggggaaggc aaaatgaagg gttgagtggt gccgttgttt tggaggcggc tccgcgctgt 1765

gcgcagtact gtgactccac ggactgttat ccctgtgtgt gctgtaggac agccccgcag 1825

tacgctgagc tccggctctc agccacggac gatgctcgct tcgtcttggt tctttttgat 1885

tttgactttg atcgcgcatt attcaccggt atttatgtgc tctttactct gtgtagttct 1945

gactgttatt ttgtagccac gctgatgcca tatagaggta actaaaacca gaaataaaag 2005

gttaaggaca aaccccatcc ccccctccct ttaccccgct accctctatc ggatctgtgc 2065

tcacagctgt tttcagacga cggaaaatcc atttaagagg aacgaatttc gcggccggtg 2125

cgaccatgaa ttgtacaacg ctttgtaaag ttcacattta cagaataata aaggaaatga 2185

tgattacaaa c                                                      2196


<210> 2
<211> 277
<212> PRT
<213> Quail coturnix coturnix japonica

<400> 2
Met Tyr Ile Ile Tyr Leu Lys Leu Tyr Gln Ala Pro Leu Lys Lys Ser
 1               5                  10                  15
Glu Arg Val Leu Arg Ala Val Pro Thr Ser Leu His Leu His Ile His
            20                  25                  30
Ala Ser Leu Tyr Pro Asp Ile Pro Thr Ser Pro His Pro Tyr Ile Thr
        35                  40                  45
Val Ser Pro His Pro Cys Ile Leu Ile Ser Pro His Ile Ser Leu Ser
    50                  55                  60
Pro His Pro His Phe Pro Ala Pro Ser Ser Cys Arg Thr Leu Leu Leu
 65                 70                  75                  80
Pro Ala Val Ser Arg Gln Gln Arg Val Pro Ser Pro Ser His Tyr Gly
```

```
                85                  90                  95
Val His Met Asp Cys Gly Leu Met Glu Pro Pro Ser Arg Glu Ser Ile
            100                 105                 110
Ala Phe Pro Asp Arg Phe Pro Ala Lys Leu Met Leu Gly Leu Leu Ile
        115                 120                 125
Phe Thr Glu Ile Thr Cys Cys Glu Arg Arg Arg Val Tyr Gly Pro Leu
    130                 135                 140
Thr Asn Pro Lys Pro Gln Ser Leu Ser Ala Ile Asn Pro Lys Gly Gly
145                 150                 155                 160
Thr Glu Asn Ser Arg Leu Phe Trp Gly Ser Asn Cys Cys Leu Glu Val
                165                 170                 175
Phe Leu Ser Tyr Arg Cys Val Thr Leu Ser Ala Gln Gly Arg Gly Thr
            180                 185                 190
Pro Lys Pro Gly Leu Ser Gly Ala Pro Cys Thr Asp Arg Arg Ala Met
        195                 200                 205
Ser Phe Phe His Cys Tyr Arg Ser Ile Leu His Thr Lys Val Gly Phe
    210                 215                 220
Tyr Val Lys Ile Lys Lys Lys Lys Arg Lys Lys Lys Arg Asn Asn Asn
225                 230                 235                 240
Asn Asn Asn Lys Asn Asn Asn Asn Lys Tyr Ile Tyr Ile Leu Lys Arg
                245                 250                 255
Lys Glu Asn Lys Ala Lys Thr Cys Asn Thr Val Phe Ser Arg Arg Asn
            260                 265                 270
Lys Ser Thr Asn Lys
        275
```

<210> 3
<211> 277
<212> PRT
<213> Quail coturnix coturnix japonica

<220>
<221> DOMAIN
<222> (6)..(27)
<223> Leucine Zipper Domain

<220>
<221> DOMAIN
<222> (227)..(249)
<223> Basic Domain

<220>
<221> SITE
<222> (230)..(235)
<223> Nuclear Localization Signal

<400> 3

```
Met Tyr Ile Ile Tyr Leu Lys Leu Tyr Gln Ala Pro Leu Lys Lys Ser
1               5                   10                  15

Glu Arg Val Leu Arg Ala Val Pro Thr Ser Leu His Leu His Ile His
            20                  25                  30

Ala Ser Leu Tyr Pro Asp Ile Pro Thr Ser Pro His Pro Tyr Ile Thr
        35                  40                  45

Val Ser Pro His Pro Cys Ile Leu Ile Ser Pro His Ile Ser Leu Ser
    50                  55                  60
```

```
Pro His Pro His Phe Pro Ala Pro Ser Ser Cys Arg Thr Leu Leu Leu
 65                 70                  75                  80

Pro Ala Val Ser Arg Gln Gln Arg Val Pro Ser Pro Ser His Tyr Gly
                85                  90                  95

Val His Met Asp Cys Gly Leu Met Glu Pro Pro Ser Arg Glu Ser Ile
            100                 105                 110

Ala Phe Pro Asp Arg Phe Pro Ala Lys Leu Met Leu Gly Leu Leu Ile
        115                 120                 125

Phe Thr Glu Ile Thr Cys Cys Glu Arg Arg Arg Val Tyr Gly Pro Leu
    130                 135                 140

Thr Asn Pro Lys Pro Gln Ser Leu Ser Ala Ile Asn Pro Lys Gly Gly
145                 150                 155                 160

Thr Glu Asn Ser Arg Leu Phe Trp Gly Ser Asn Cys Cys Leu Glu Val
                165                 170                 175

Phe Leu Ser Tyr Arg Cys Val Thr Leu Ser Ala Gln Gly Arg Gly Thr
            180                 185                 190

Pro Lys Pro Gly Leu Ser Gly Ala Pro Cys Thr Asp Arg Arg Ala Met
        195                 200                 205

Ser Phe Phe His Cys Tyr Arg Ser Ile Leu His Thr Lys Val Gly Phe
    210                 215                 220

Tyr Val Lys Ile Lys Lys Lys Lys Arg Lys Lys Lys Arg Asn Asn Asn
225                 230                 235                 240

Asn Asn Asn Lys Asn Asn Asn Asn Lys Tyr Ile Tyr Ile Leu Lys Arg
                245                 250                 255

Lys Glu Asn Lys Ala Lys Thr Cys Asn Thr Val Phe Ser Arg Arg Asn
            260                 265                 270

Lys Ser Thr Asn Lys
        275


<210> 4
<211> 834
<212> DNA
<213> Quail coturnix coturnix japonica

<220>
<221> CDS
<222> (1)..(834)
<223> Proliferation Arrest Transcription Factor

<220>
<221> misc_feature
<222> (15)..(78)
<223> Leucine Zipper Domain

<220>
<221> misc_feature
<222> (678)..(744)
```

<223> Basic Domain

<220>
<221> misc_feature
<222> (687)..(702)
<223> Nuclear Localization Signal

<400> 4

```
atg tat att ata tat cta aag tta tac caa gca cct tta aag aaa tca     48
Met Tyr Ile Ile Tyr Leu Lys Leu Tyr Gln Ala Pro Leu Lys Lys Ser
  1               5                  10                  15

gag cgc gtg ctc cgt gca gtc ccc aca tcc cta cat ctc cat atc cat     96
Glu Arg Val Leu Arg Ala Val Pro Thr Ser Leu His Leu His Ile His
             20                  25                  30

gca tcc cta tat cct gac atc cct aca tca ccg cat ccc tat atc act    144
Ala Ser Leu Tyr Pro Asp Ile Pro Thr Ser Pro His Pro Tyr Ile Thr
         35                  40                  45

gta tct cca cat ccc tgt atc ctc ata tcc cca cat atc tcc tta tcc    192
Val Ser Pro His Pro Cys Ile Leu Ile Ser Pro His Ile Ser Leu Ser
     50                  55                  60

cca cac ccc cat ttc cca gct ccc agc agc tgt agg acc ctc ctc ctc    240
Pro His Pro His Phe Pro Ala Pro Ser Ser Cys Arg Thr Leu Leu Leu
 65                  70                  75                  80

cca gct gtg tcc cgg cag cag cgg gtg ccg tcc cca tcc cac tat ggg    288
Pro Ala Val Ser Arg Gln Gln Arg Val Pro Ser Pro Ser His Tyr Gly
                 85                  90                  95

gtg cac atg gac tgt ggc ctc atg gag cca ccg agc agg gaa tcg att    336
Val His Met Asp Cys Gly Leu Met Glu Pro Pro Ser Arg Glu Ser Ile
            100                 105                 110

gcc ttt cct gat cgt ttt cct gct aag ctt atg ttg gga ttg ctt ata    384
Ala Phe Pro Asp Arg Phe Pro Ala Lys Leu Met Leu Gly Leu Leu Ile
        115                 120                 125

ttt aca gaa ata act tgc tgt gag cgg aga agg gtg tat ggc cca tta    432
Phe Thr Glu Ile Thr Cys Cys Glu Arg Arg Arg Val Tyr Gly Pro Leu
    130                 135                 140

acc aac cca aaa ccc cag agc ctc tcg gct ata aat cct aaa ggc ggc    480
Thr Asn Pro Lys Pro Gln Ser Leu Ser Ala Ile Asn Pro Lys Gly Gly
145                 150                 155                 160

aca gag aac tca cgt ctc ttc tgg ggc tcc aac tgc tgt tta gaa gtg    528
Thr Glu Asn Ser Arg Leu Phe Trp Gly Ser Asn Cys Cys Leu Glu Val
                165                 170                 175

ttc ctg tcg tat aga tgt gtc acg ttg tca gcc cag ggt cgg ggc acc    576
Phe Leu Ser Tyr Arg Cys Val Thr Leu Ser Ala Gln Gly Arg Gly Thr
            180                 185                 190

cca aag cca ggg ctg tca gga gct ccg tgc acc gat agg cgt gcc atg    624
Pro Lys Pro Gly Leu Ser Gly Ala Pro Cys Thr Asp Arg Arg Ala Met
        195                 200                 205

agt ttc ttc cac tgt tat aga agc att ttg cac acc aag gtt ggt ttt    672
```

```
Ser Phe Phe His Cys Tyr Arg Ser Ile Leu His Thr Lys Val Gly Phe
    210                 215                 220

tat gtc aaa att aag aag aaa aaa aga aaa aaa aaa aga aat aat aat    720
Tyr Val Lys Ile Lys Lys Lys Lys Arg Lys Lys Lys Arg Asn Asn Asn
225                 230                 235                 240

aat aat aat aaa aat aat aat aat aaa tat ata tat ata tta aaa aga    768
Asn Asn Asn Lys Asn Asn Asn Asn Lys Tyr Ile Tyr Ile Leu Lys Arg
                245                 250                 255

aaa gaa aac aaa gcc aaa act tgc aat act gtt ttt agc aga cgg aat    816
Lys Glu Asn Lys Ala Lys Thr Cys Asn Thr Val Phe Ser Arg Arg Asn
            260                 265                 270

aaa tcc acg aac aaa taa                                             834
Lys Ser Thr Asn Lys
        275


<210> 5
<211> 277
<212> PRT
<213> Quail coturnix coturnix japonica

<400> 5
Met Tyr Ile Ile Tyr Leu Lys Leu Tyr Gln Ala Pro Leu Lys Lys Ser
 1               5                  10                  15
Glu Arg Val Leu Arg Ala Val Pro Thr Ser Leu His Leu His Ile His
            20                  25                  30
Ala Ser Leu Tyr Pro Asp Ile Pro Thr Ser Pro His Pro Tyr Ile Thr
        35                  40                  45
Val Ser Pro His Pro Cys Ile Leu Ile Ser Pro His Ile Ser Leu Ser
    50                  55                  60
Pro His Pro His Phe Pro Ala Pro Ser Ser Cys Arg Thr Leu Leu Leu
65                  70                  75                  80
Pro Ala Val Ser Arg Gln Gln Arg Val Pro Ser Pro Ser His Tyr Gly
                85                  90                  95
Val His Met Asp Cys Gly Leu Met Glu Pro Pro Ser Arg Glu Ser Ile
            100                 105                 110
Ala Phe Pro Asp Arg Phe Pro Ala Lys Leu Met Leu Gly Leu Leu Ile
        115                 120                 125
Phe Thr Glu Ile Thr Cys Cys Glu Arg Arg Arg Val Tyr Gly Pro Leu
    130                 135                 140
Thr Asn Pro Lys Pro Gln Ser Leu Ser Ala Ile Asn Pro Lys Gly Gly
145                 150                 155                 160
Thr Glu Asn Ser Arg Leu Phe Trp Gly Ser Asn Cys Cys Leu Glu Val
                165                 170                 175
Phe Leu Ser Tyr Arg Cys Val Thr Leu Ser Ala Gln Gly Arg Gly Thr
            180                 185                 190
Pro Lys Pro Gly Leu Ser Gly Ala Pro Cys Thr Asp Arg Arg Ala Met
        195                 200                 205
Ser Phe Phe His Cys Tyr Arg Ser Ile Leu His Thr Lys Val Gly Phe
    210                 215                 220
Tyr Val Lys Ile Lys Lys Lys Lys Arg Lys Lys Lys Arg Asn Asn Asn
225                 230                 235                 240
Asn Asn Asn Lys Asn Asn Asn Asn Lys Tyr Ile Tyr Ile Leu Lys Arg
                245                 250                 255
Lys Glu Asn Lys Ala Lys Thr Cys Asn Thr Val Phe Ser Arg Arg Asn
            260                 265                 270
Lys Ser Thr Asn Lys
```

275

<210> 6
<211> 654
<212> DNA
<213> Quail coturnix coturnix japonica

<220>
<221> misc_feature
<222> (1)..(654)
<223> fragment inserted in the RCAS vector for antisense strategy

<400> 6
```
tcgatttgta tgcggctttc ctgagtgcct tagaatctct tgatctcttt tgttccttct 60
tttgttgtcg gtttggtttg gtttgggttt ttgttgggtt ttcttttttc ttttccaagg 120
aaaacagatg gagagaatca gctcataacc acctccttcc tcctctttgt ttacgatact 180
attttaaatt atagaattcg gggctgcttt ataatgtgaa taggaaactg cgttcatcta 240
ggtctgatag tgtaaaatca gagcatcagt atttatggta tgtctgcacc tttattccta 300
tgcttgacta ttagcaatat tacatgtata ttatatatct aaagttatac caagcacctt 360
taaagaaatc agagcgcgtg ctccgtgcag tccccacatc cctacatctc catatccatg 420
catccctata tcctgacatc cctacatcac cgcatcccta tatcactgta tctccacatc 480
cctgtatcct catatcccca catatctcct tatccccaca cccccatttc ccagctccca 540
gcagctgtag gaccctcctc ctcccagctg tgtcccggca gcagcgggtg ccgtccccat 600
cccactatgg ggtgcacatg gactgtggcc tcatggagcc accgagcagg gaat       654
```

<210> 7
<211> 20
<212> DNA
<213> Artificial Sequence

<220>
<223> PCR primer

<400> 7
```
tcctatgctt gactattagc                                             20
```

<210> 8
<211> 19
<212> DNA
<213> Artificial sequence

<220>
<223> PCR primer

<400> 8
```
tacagctgct gggagctgg                                              19
```

<210> 9
<211> 24
<212> DNA
<213> Artificial Sequence

<220>
<223> PCR product

<220>
<223> Description of Artificial Sequence:oligo

<400> 9
cctgacatcc ctacatcacc gcat 24

<210> 10
<211> 18
<212> DNA
<213> Artificial Sequence

<220>
<223> PCR primer

<220>
<223> Description of Artificial Sequence:oligo

<400> 10
tggttctcca ttcctgag 18

<210> 11
<211> 18
<212> DNA
<213> Artificial Sequence

<220>
<223> PCR primer

<220>
<223> Description of Artificial Sequence:oligo

<400> 11
aggacgtact aggaacag 18

<210> 12
<211> 18
<212> DNA
<213> Artificial Sequence

<220>
<223> PCR primer

<220>
<223> Description of Artificial Sequence:oligo

<400> 12
agctgatgga tggtgaag 18

<210> 13
<211> 18
<212> DNA
<213> Artificial Sequence

<220>
<223> PCR primer

<220>

<223> Description of Artificial Sequence:oligo

<400> 13
aatactcctc tgctgagg 18

<210> 14
<211> 22
<212> DNA
<213> Artificial Sequence

<220>
<223> PCR primer

<220>
<223> Description of Artificial Sequence:oligo

<400> 14
atccgttgtg gatctgacat gc 22

<210> 15
<211> 22
<212> DNA
<213> Artificial Sequence

<220>
<223> PCR primer

<220>
<223> Description of Artificial Sequence:oligo

<400> 15
tgtcattgag agcaatgcca gc 22

<210> 16
<211> 21
<212> DNA
<213> Artificial Sequence

<220>
<223> EMSA oligo

<220>
<223> Description of Artificial Sequence:oligo

<400> 16
ctaggatatt gcgcaatatg c 21

<210> 17
<211> 19
<212> DNA
<213> Artificial Sequence

<220>
<223> EMSA oligo

<220>
<223> Description of Artificial Sequence:oligo

<400> 17
tttctgaagt cagtcagcc 19

<210> 18
<211> 22
<212> DNA
<213> Artificial Sequence

<220>
<223> EMSA oligo

<220>
<223> Description of Artificial Sequence:oligo

<400> 18
tagttttcgc gcttaaattt ga 22

<210> 19
<211> 255
<212> DNA
<213> Quails coturnix coturnix japonica

<400> 19

tcctatgctt gactattagc aatattacat gtatattata tatctaaagt tataccaagc 60

acctttaaag aaatcagagc gcgtgctccg tgcagtcccc acatccctac atctccatat 120

ccatgcatcc ctatatcctg acatccctac atcaccgcat ccctatatca ctgtatctcc 180

acatccctgt atcctcatat ccccacatat ctccttatcc ccacaccccc atttcccagc 240

tcccagcagc tgtag

<210> 20
<211> 75
<212> PRT
<213> Quails coturnix coturnix japonica

<223> Description of Artificial Sequence:oligo

<400> 20

```
Met Tyr Ile Ile Tyr Leu Lys Leu Tyr Gln Ala Pro Leu Lys Lys Ser
  1               5                  10                  15
Glu Arg Val Leu Arg Ala Val Pro Thr Ser Leu His Leu His Ile His
             20                  25                  30
Ala Ser Leu Tyr Pro Asp Ile Pro Thr Ser Pro His Pro Tyr Ile Thr
         35                  40                  45
Val Ser Pro His Pro Cys Ile Leu Ile Ser Pro His Ile Ser Leu Ser
     50                  55                  60
Pro His Pro His Phe Pro Ala Pro Ser Ser Cys
 65                  70
```

<210> 21
<211> 10
<212> peptide
<213> Quails coturnix coturnix japonica

<400> 21

vtlsaqgrgt 10

<210> 22
<211> 10
<212> peptide
<213> Quails coturnix coturnix japonica

<400> 21

ygpltnpkpq 10

<210> 23
<211> 10
<212> peptide
<213> Quails coturnix coturnix japonica

<400> 23

rtlllpavsr 10

<210> 24
<211> 654
<212> DNA
<213> Artificial Sequence

<220>
<223> Description of Artificial Sequence: antisens of SEQ ID N°6

<400> 24

taagggacga gccaccgagg tactccggtg tcaggtacac gtggggtatc accctacccc 60

tgccgtgggc gacgacggcc ctgtgtcgac cctcctcctc ccaggatgtc gacgaccctc 120

gaccctttac ccccacaccc ctattcctct atacacccct atactcctat gtccctacac 180

ctctatgtca ctatatccct acgccactac atccctacag tcctatatcc ctacgtacct 240

atacctctac atccctacac ccctgacgtg cctcgtgcgc gagactaaag aaatttccac 300

gaaccatatt gaaatctata tattatatgt acattataac gattatcagt tcgtatcctt 360

atttccacgt ctgtatggta tttatgacta cgagactaaa ttgtgatagt ctggtcatac 420

ttgcgtcaaa ggataagtgt aatatttcgt cggggcttaa gatattaaat tttatcatag 480

catttgtttc tcctccttcc tccaccaata ctcgactaag agaggtagac aaaaggaacc 540

```
ttttcttttt tcttttgggt tgtttttggg tttggtttgg tttggctgtt gttttcttcc  600
ttgttttctc tagttctcta agattccgtg agtcctttcg gcgtatgttt agct        654
```

## Description

The present invention relates to new compounds useful for controlling cell proliferation and/or differentiation, and to biological applications thereof.

Different compounds have been described in the prior art as enabling the positive or negative control of cell proliferation and/or differentiation. Such compounds namely comprise transcription factors and modulators, among which different families can be distinguished : E2Fs, DPs, pRB, p107, p130, cyclins, cyclin dependent kinases, cyclin dependent kinase inhibitors. The E2F and DP transcription factor families are essential for regulating the correct timing of several genes involved in control of cell proliferation. Heterodimers composed of an E2F family member and a DP family member form stable trimeric complexes with retinoblastoma proteins family (pRB, p130, p107). Disruption of the interactions between pRB family protein and E2F promotes cell proliferation and conversly, the stabilization of these interactions inhibits growth. E2F1 / 2 / 3 bind to cyclin A and not E2F 4/5. Cell cycle progression in eukaryotic cells is governed by interactions between Cyclins (A, B, D and E), cyclin dependent kinases (Cdk 1/2/4 and 6) and cyclin dependent kinase inhibitors (CDKI). The activities of Cdks are further modulated by phosphorylation and dephosphorylation of specific residues, and by the CDKIs. CDKIs are classified into two families : the Cip/Kip family members (P21, P27, and P57) which possess the ability to inhibits a variety of cyclin-Cdk complexes, and the Ink4 family members (P16, P15, P18, and P19) which are Cdk4/Cdk6-specific inhibitors.

The present invention now provides with new compounds capable of controlling cell proliferation and/or differentiation. It namely relates to isolated polypeptides which are capable of inhibiting the proliferation of a cell population, and/or of stimulating the differentiation of a cell population, and/or of stimulating the establishment of a quiescent state in a cell population.

They are characterized in that they :

- comprise at least one sequence of the leucine zipper domain type at the N-terminus,
- comprise at least one sequence of the basic domain type at the C-terminus, and
- comprise at least one sequence of the nuclearization signal type and/or is coupled to a compound capable of performing a nuclearization of said polypeptide into at least one cell of said population.

Such polypeptides advantageously comprise at least one phosphorylation site. The polypeptides of the invention substantially show the characteristic of transcription factors.

In the present application, the expression "inhibiting" is meant as negatively controlling, and herein also includes blocking. Similarly, the expression "stimulating" is meant as positively controlling, and herein also includes inducing. Assessment of a negative or a positive control is of common knowledge to the skilled persons, it is understood as a statistically significant difference observed between a test treatment and a control treatment.

The polypeptides of the invention can be used for a wide variety of cells. Said cell population may comprise cells chosen among the group consisting of animal cells, vertebrate cells, bird cells, mammal cells, rodent cells, human cells, CNS cells, cells from heart, cells from brain, cells from cerebellum, cells from retina, nerve cells, neuronal cells, axonal cells, cells from a fibrous tissue, cells from a connective tissue, epithelial cells, fibroblats, cells from liver, from spleen, from thymus, and any cell which can be considered as a progeny cell or a descendent cell thereof.

Said cell population may comprise a cell population chosen among the group consisting of normal cells and outstandingly also hyperproliferative cells such as pathologically hyperproliferative cells (tumoral cells).

The polypeptides of the invention are particularly notable for being capable of inhibiting the proliferation, and/or of stimulating the differentiation, and/or of stimulating the establishment of a quiescent state in pathological cells such as human or animal tumoral cells, and particularly neuroblastoma cells, retinoblastoma cells, pheochromocytoma cells, cells from human or animal breast, colon, kidney, vesica, prostate tumors.

Method for assessing proliferation/differentiation/quiescent states are available and know to be skilled persons. Indeed, blocking polypeptide activity (*e.g.* via blocking its nuclearization) or blocking polypeptide expression (*e.g.* via blocking the transcription of its DNA) leads to additional mitotic cycles (additional mitosis and/or additional S phases) and/or stimulation of transcription of genes involved in proliferation and/or inhibition of genes involved in differentiation or quiescence.

In the present application, "proliferation" refers to an increase in cell number, and is herein also meant as the pursuit of mitotic cycles: it can be assessed via measurement of the number of S phases, and/or of the number of mitosis (M phases) performed by said cell population.

The establishment of a quiescent state in a cell population can be assessed by the skilled persons namely *via* observation of the establishment of the G1 and/or G0 mitotic cycle state(s) in said cell population.

The establishment of a differentiation state can be assessed by the skilled persons by observing the morphological and/or cytological characteristics of said the cell population, and/or the presence or absence of the expression of differentiation markers.

When reference is made in the present application to a compound "capable of" showing a biological effect, it is herein meant that this capacity can be observed by the skilled person when said compound is assayed under experimental conditions of the physiological type, *i.e. in vivo* conditions, or *in vitro* conditions mimiking *in vivo* conditions (typically, when an animal cell is involved in such an assay, such conditions comprise the choice of one or several media of which respective pH and/or composition are appropriate for testing the existence or the absence of said capacity); it is also understood that the skilled person can find without undue burden a quantity of compound appropriate for performing said assay.

By "sequence of the basic domain type", it is herein meant a sequence rich in basic amino acids which is capable of binding to a DNA molecule under physiological conditions.

By "sequence of the leucine zipper domain type", it is herein meant a sequence comprising a Leucine Zipper pattern. A Leucine Zipper pattern consists of a periodical repetition of leucine residues at every seventh position over a distance convering eight helical turns. The leucine side chains extending from one alpha helix interact with those from a similar alpha helix of a second polypeptide, facilitating dimerization. The present consensus pattern is :

L-X (6) -L- X (6) - L- (6) - L.

By "sequence of the nuclearization signal type" it is herein meant a sequence comprising a nuclearisation signal. A nuclearization signal is a short sequence of amino acid of arginin and lysin required for nuclear localization, such as KKKRR(K).

A polypeptide of the invention, when isolated from natural source in a normal physiological state is capable of autonuclearization (it comprises a sequence of the nuclearization signal type), but the skilled person can alternatively choose, when appropriate, to couple the polypeptide to any other nuclearization compound, or to replace the nuclearization signal region of said polypeptide with such another nuclearization compound. A polypeptide of the invention may also be produced by incubation of genetically engineered cells and/or synthesis.

The polypeptides of the invention may advangeously be capable under physiological conditions of binding to a DNA of the nuclear type, notably to oligonucleotides representing DNA binding site, and in particular to the E2F binding site (5'- TAGTTTTCGCGCTTAAATTTGA-3'). The polypeptides of the invention do not bind to C/EBP or to the AP1 binding site (see example 1 below).

According to a particular aspect of the invention, the polypeptides of the invention may advantageously be capable of forming complexes with one or several compound(s), notably transcription factor(s) or modulator(s), characteristic of mitosis termination or of a post-mitotic state (differentiation, quiescence), such as E2F-4, p130, DP-1, DP-2.

Advantageously, they do not bind under physiological conditions to compound(s), notably transcription factor (s) or modulator(s), which are positively involved in mitotic cycle progression or continuation such as E2F-1, E2F-2, E2F-3.

Another remarquable feature of the polypeptides according to the invention is that they may be capable when expressed in the nucleus of a cell, of:

stimulating the level of compound(s), notably of protein(s) such as transcription factor(s) or modulator(s), positively involved in mitosis termination or of a post-mitotic state (differentiation, quiescence), such as E2F-4, p130, present in said cell, and/or of

inhibiting the level of compound(s), notably of protein(s), such as transcription factor(s) or modulator(s), positively involved in mitotic cycle progression or continuation such as E2F-1, E2F-2, E2F-3, present in said cell, and/or of inhibiting the transcription of genes positively involved in mitotic cycle progression or continuation (mitosis, DNA synthesis).

An outstanding feature of the polypeptides according to the invention is that when their nuclearization is blocked, additional mitotic cycles (mitosis, S phases), and/or stimulation of transcription of genes involved in proliferation and/ or inhibition of genes involved in differentiation or quiescence occur.

The present invention also encompasses any isolated polypeptide such as obtained by isolation from human or animal cell, *e.g.* a neuroretina cell, of a polypeptide of which apparent molecular weight is of about 50 kDa when measured by Western blot analysis

It particularly encompasses isolated polypeptide of which sequence corresponds to a sequence chosen among the group consisting of SEQ ID N°2, SEQ ID N°3, SEQ ID N°5, and any polypeptidic sequence, namely any human polypeptide sequence, such as obtained by conservative substitution and/or deletion and/or inversion of aminoacids of SEQ ID N°2, SEQ ID N°3, SEQ ID N°5.

SEQ ID N°2, N°3, N°5 all correspond to the same polypeptidic sequence (*cf.* figure 1B), named PATF (Proliferation Arrest Transcription Factor) by the inventors. It has been isolated from quail normal neuroretina cells. It comprises a leucine zipper domain (position 6-27; L-X (6) -L- X (6) - L- (6) - L) at the N-terminus, a basic domain (position 227-249) at the C terminus, and a nuclearization signal (position 230-235; KKKRR(K)). It advantageously further comprises phosphorylation sites, and appears as a bZIP family member. It shows no homology with any known product, as assessed on data banks.

Polypeptides such as obtained by conservative substitution and/or deletion and/or inversion of aminoacids of SEQ ID N°2, SEQ ID N°3, SEQ ID N°5 will be refered to as "variant" polypeptides. A substitution/deletion/inversion operation, or a series of substitution/deletion/inversion operations is herein considered as conservative when the polypeptide thus obtained is still capable (at least on a qualitative point of view, but not necessarily on a quantitative point of view) of showing at least one of the biological functions shown by the "parent" polypeptide, *e.g.* inhibiting proliferation, and/or stimulating differentiation, and/or stimulating the establishment of a quiescent state in a cell population, as above-described and as further illustrated in the below examples. The word "deletion" herein encompasses fragments of a parent polypeptide. The present invention more particularly encompasses such polypeptides of which sequence shows an homology of at least 50%, particularly of at least 60%, notably of at least 70%, preferably of at least 80%, most preferably of at least 90% with SEQ ID N°2, N°3 or N°5, and which is also capable (at least on a qualitative point of view) of inhibiting proliferation, and/or of stimulating differentiation, and/or of stimulating the establishment of a quiescent state in a cell population, as above-described and as further illustrated in the below examples. Examples of such variant polypeptides notably comprise the human equivalents of SEQ ID N°2, 3, 5, and the conservative variants, notably fragments, thereof. The teaching of the present invention allows the skilled persons to isolate and sequence such human equivalents (see example 2 below) ; they can be isolated *e.g.* by extracting the polypeptidic population of human normal cells, screening them with a monoclonal antibody of the invention (described below and illlustrated in the below examples), or by extracting the RNA population of normal human cells and screening them with SEQ ID N°1 (complete quail cDNA), SEQ ID N°4 (quail ORF), or any appropriate probe or primer derived from a polypeptide of the invention (example of such probes - SEQ ID N°6 of 654 bp, SEQ ID N°19 of 255 bp - and of such primers -SEQ ID N°7-8, 10-11, 12-13, 14-15- are given and illustrated below), and cloning and sequencing those reactive clones.

The present invention also encompasses any polypeptide of which sequence shows with a sequence chosen among the group consisting of SEQ ID N°2, N°3, N°5 an homology of at least 80%, notably of at least 90%, but which is not capable of inhibiting proliferation, and/or of stimulating differentiation, and/or of stimulating the establishment of a quiescent state in a cell population, as above-described and as further illustrated in the below examples. Such polypeptides are herein refered to as "mutant polypeptides", and notably comprise non functional mutants. Modes of obtention of such mutants are of common knowledge to the skilled persons; they can *e.g.* be obtained *via* deleting or introducing a mutation in the leucine zipper and/or the basic domain, and/or in the nuclearization signal of the "parent" polypeptide.

The present invention also provides with isolated peptides chosen among the group consisting of SEQ ID N°21, N°22, N°23. These peptides have been chosen in the region of the polypeptides of the invention which is between the leucine zipper domain and the basic domain of SEQ ID N°2, N°3, N°5. They allow the production of products which are capable of binding to a polypeptide according to the invention, and notably with the production of purified serum and of monoclonal antibodies. They remarquably allow the production of serum and antibody which bind to the polypeptides of the invention without binding to any known transcription factors. These peptides do not show any significant homology on data banks, and have the advantage of being immunogenic (see example 2 below). They could thus by used as vaccinal agents. The present invention also encompasses the isolated polynucleotides coding for or corresponding to peptides SEQ ID N°21, 22, 23.

According to another aspect of the present invention, there is provided products which are capable of (under physiological conditions, as above-described) binding to at least one a polypeptide according to the invention. These products will be refered to herein as "binding products". Methods for assessing whether a product binds or not to a given polypeptide are of common knowledge to the skilled persons; they may *e.g.* include the use of cytometry and/or immunoprecipitation and/or western blotting, and/or competition assays. Examples of such procedures are given in the below examples (see *e.g.* example 1, "immunoprecipitation and western blotting").

Such products may notably be chosen among the group consisting of solutions of the serum type, solutions of the purified serum type, compounds of the antibody type, compounds of the monoclonal antibody type, fragments of such antibodies, notably Fab, F(ab')2, CDR fragments in so far these fragments have retained the capacity to bind to a polypeptide according to the invention, *e.g.* when assayed by themselves or grafted into another antibody type structure, and humanized antibodies such as obtained by grafting such fragments into an antibody type structure. The polypeptides of the invention indeed allow the production of serum or of antibody which binds to them. Prefered binding products do bind to a polypeptide/peptide/mutant peptide of the invention without binding (under comparable experimental conditions) to other known transcription factor(s) or transcription modulator(s), and notably without binding to any member of the E2F family (see example 2 below).

The invention also provides with products which are capable of binding with the mutant polypeptides of the invention.

The binding products of the invention can be used for isolating variant and mutant polypeptides according to the invention. The present invention thus encompasses any polypeptide such as obtained by isolation from a cell with the help of a binding product of the invention, and notably with a serum or an antibody of the invention (see *e.g.* example 3 below).

The binding products of the invention can also be used as detection reagent, and/or as biologically active principles. For detection purposes, they can be, when appropriate or found convenient, coupled to compounds making detection easier, for example they can be coupled to a fluorescence element such as FITC, or to a radioactive element.

Alternatively to such couplings, the use of binding products according to the invention can be associated with the use of secondary reagent(s) which bind(s) to them, and which themselves bear(s) compounds making detection easier.

The binding products of the invention can also be used as biologically active principle, notably binding products which are capable of inhibiting or blocking the biological activity of their target polypeptide, and those which are capable of stimulating the biological activity of their target polypeptide.

Those binding products which can inhibit or block the biological activity of their target polypeptide are capable of inducing or stimulating additional S phases and/or additional mitosis, and/or of inducing or stimulating entry into a mitotic cycle, and/or of inhibiting and/or blocking differentiation. Such binding products of inhibiting activity notably comprise those binding products of the invention which bind to a nuclearization signal region of a polypeptide of the invention.

Those binding products of the invention which stimulate the biological activity of their target polypeptide are capable of inhibiting or blocking additional S phases and/or additional mitosis, and/or of stimulating and/or inducing the exit from a mitotic cycle, and/or of stimulating proliferation, and/or of stimulating differentiation. Such binding products notably comprise those which stabilize the formation of complexes involving a polypeptide of the invention, such as complexes between a polypeptide of the invention and transcription factor/modulator and/or a DNA molecule.

Discriminating between a binding product of inhition activity and a binding product of stimulation activity can be made by routine basic procedures, such as those commonly used to assay whether a test compound induces or stops mitosis, proliferation or differentiation.

The polypeptides of the invention also allow the obtention of compound comprising them. The polypeptides of the invention notably allows the obtention of isolated complexes comprising at least one polypeptide according to the invention. Among those complexes, the present invention particularly encompasses those which are heteromeric. Particular complexes of the invention are dimeric or trimeric heteromeric complexes. Useful complexes of the invention comprise those which comprise at least one polypeptide of the invention and at least one product chosen among the group consisting of the transcription factors and the transcription modulators, such as E2F-4, E2F-5, DP-1, DP-2, pRB, p130, p107, the polynucleotidic molecules such as any DNA molecule, and particularly any nuclear DNA molecule.

Preferred DNA molecules of the complexes of the invention include those which are therapeutically useful, notably in antioncogeny, such as p53.

The polypeptides of the invention are also useful as stabilization agents. Indeed, they are capable of stabilizing an interaction between a DNA molecule and a transcription factor/modulator, and thus stimulating the formation of complexes as above-described.

According to another aspect of the present invention, there is provided isolated polynucleotides of which transcription or translation is downregulated when a cell in terminal mitosis, in quiescent phase or in differentiation is induced to proliferate. The present invention notably encompasses isolated polynucleotides which are characteristic of a post-mitotic state. By a polynucleotide "characteristic of a post-mitotic state", it is herein intended a polynucleotide which is present in quiescent and/or differentiating cells and which is absent in proliferating cells. Such polynucleotides can be obtained *e.g. via* autosubstracted cDNA library.

The isolated polynucleotides of the invention are capable, *via* their transcription or their translation, of inhibiting the proliferation of a cell population and/or of stimulating the differentiation of a cell population and/or of stimulating the establishment of a quiescent state in a cell population. The meaning intended for the expressions "capable of", "inhibiting", "stimulating", "proliferation", "differentiation", "quiescent state" is as defined above in the polypeptide section, and is further illustrated in the below examples.

Such polynucleotides comprises RNA, mRNA, cDNA and DNA.

They are characterized in that their respective sequence corresponds to a sequence coding for a polypeptide comprising:

- at least one sequence of the leucine zipper type at the N-terminus,
- at least one sequence of the basic domain type at the C-terminus, and
- at least one sequence of the nuclearization signal type.

  The polynucleotides of the invention notably comprises those of which sequence codes for a polypeptide of the invention, including variant polypeptides and human variant polypeptides.

  By "coding for", it is herein made reference to the genetic universal code with its redundancies.

The present invention encompasses any polynucleotide of which sequence appears as complementary, according to the genetic code and its redundancies, to a polynucleotide of the invention.

The polynucleotides of the invention particularly comprise

- those of which sequence corresponds to a sequence coding for SEQ ID N°2, 3, 5 and their human variant homologues,
- those of which sequence shows an homology of at least 50%, particularly of at least 60%, notably of at least 70%, preferably of at least 80%, most preferably of at least 90% with SEQ ID N°1 (2196 bp), or SEQ ID N°4 (ORF of 834 bp) while retaining the corresponding biological activity of the encoded product, as above-defined,
- those of which sequence corresponds to SEQ ID N°1 or SEQ ID N°4. The mRNA corresponding to SEQ ID N°1 and SEQ ID N°4 or SEQ ID N°2, 3, 5 (polypeptide) represent about 6,8-7 kb. This mRNA (see example 1 below) can be used to isolate mRNA homologues from cells of a species other than quails, notably from human cells, and for subsequenty deducing the DNA and polypeptidic sequence of this homologue.

The present invention also encompasses any fragment of a polynucleotide of the invention which can be considered as characteristic of this polynucleotide when compared to at least one, preferably any other polynucleotides coding for an other known transcription factor(s)/modulator(s). It notably provides with such a polynucleotide fragment of which sequence corresponds to a sequence chosen among the group consisting of SEQ ID N°6 (654 bp corresponding to position 429-1082 of SEQ ID N°1), SEQ ID N°9, and SEQ ID N°19 (255 bp), SEQ ID N°7, SEQ ID N°8, SEQ ID N°10, SEQ ID N°11, SEQ ID N°12, SEQ ID N°13, SEQ ID N°14, SEQ ID N°15, SEQ ID N°21, SEQ ID N°22, SEQ ID N°23. Such a sequence is particularly useful as a probe capable of detecting/isolating a polynucleotide according to the invention without confusion with polynucleotides coding for other known transcription factors.

The present invention thus encompasses any polynucleotide to which such a probe, and in particular SEQ ID N°19 and SEQ ID N°6, binds under physiological conditions, and/or which is such as obtained via isolation with such a probe.

According to a further aspect of the invention, there is provided polynucleotides which are the antisens of the polynucleotides of the invention. Such antisens polynucleotides are particularly useful for inhibiting the activity of a polynucleotide or a polypeptide of the invention, and notably for stimulating the proliferation of a cell population, and/or for inhibiting the differentiation of a cell population, and/or for inhibiting the establishment of a quiescent state in a cell population. They can be used for genetic therapy. Such antisens polynucleotides preferably overlap the initiation codon of the target polynucleotide (e.g. ATG, see the below example 1 and also figure 1). Examples of such antisens polynucleotide comprise those of which sequence corresponds to the antisens of SEQ ID N°6 or SEQ ID N°19. The antisens of SEQ ID N°6 is shown (in anti-sens orientation) in SEQ ID N°24. Such antisens polynucleotides are particularly useful for insertion into transfection vectors so as to transfect a target cell and produce the corresponding antisens RNAs. Such RNAs advantageously inhibit the translation of compounds involved in the control of cell proliferation and/or differenciation.

The present invention also provides with detection or isolation reagents, such as probes and primers, which derive from the polynucleotides of the invention. It particularly provides with probes useful for detecting or isolating a polynucleotide according to the invention which comprise those chosen among the group consisting of SEQ ID N°1, N°4, N°6, SEQ ID N°9, SEQ ID N°19 (255 bp from position 724 to position 978 on SEQ ID N°1), SEQ ID N°7, 8, 10 11, 12, 13, 14, 15.

It also provides with couples of primers, useful for detection or isolation of a polynucleotide according to the invention *via* PCR or RT-PCT amplification, which are chosen among the group consisting of SEQ ID N°7 and 8, SEQ ID N°10 and 11, SEQ ID N°12 and 13, SEQ ID N°14 and 15.

The present invention also encompasses any isolated polynucleotide which is such as obtained by:

screening a polynucleotidic library, and in particular of cDNA or mRNA library, with at least one probe or one polynucleotide according to the invention, and/or

amplifying a polynucleotidic population with at least one primer couple of the invention.

The present invention also encompasses the ORF corresponding to such an isolated polynucleotide.

Amplification procedures such as PCR or RT-PCR are of common knowledge to the skilled persons, they can be adjusted for each case. Examples of appropriate conditions are given in the below example 1.

The skilled persons will understand that the polynucleotidic products of the invention are themselves means for isolating a polypeptide of the invention. Indeed, the present invention also encompasses those polypeptides of which sequence is such as obtained by:

i. screening a polynucleotidic library, and in particular of cDNA or mRNA library, with at least one probe or one polynucleotide according to the invention, sequencing the ORF corresponding to the polynucleotide detected by said probe or polynucleotide, and/or

amplifying a polynucleotidic population with at least one primer couple of the invention, sequencing the ORF corresponding to the amplified products,
and

ii. deducing according to the universal genetic code the polypeptidic sequence corresponding to the identified ORF.

The present invention also encompasses those polynucleotides of which sequence codes for a mutant polypeptide of the invention, or for a complex according to the invention.

Useful products derived from the polynucleotidic products of the invention comprise transfection vectors which comprise at least one polynucleotide of the invention, or at least one polynucleotide coding for a complex of the invention or a least one antisens polynucleotide of the invention. Such transfection vectors are useful in so far they allow the transfection of the polynucleotide into an animal cell, a human cell, a plant cell, or of a protist cell so as to enable a genetic medical treatment. A transfection vector which comprises a polynucleotide of the invention or a polynucleotide coding for a complexe of the invention is useful for inhibiting the proliferation of a cell population, and/or for stimulating the differentiation of a cell population, and/or for stimulating the establishment of a quiescent state in a cell population. A transfection vector which comprises an antisens polynucleotide of the invention is useful for inducing or stimulating the reversed effects. Preferred vectors comprise said at least one polynucleotide in a coding position. Most preferred vectors also comprise a promotor which allows the targetting of a particular cell type, for example the cell population to be treated. Appropriate vectors include retroviral vectors such as the RCAS vector (see the below example 1). The present invention encompasses such transfection vectors.

It also encompasses any cell which has been transfected by a polynucleotide of the invention, a polynucleotide coding for a complex of the invention an antisens polynucleotide of the invention, or a (sens or antisens) transfection vector of the invention. Transfection is herein meant as transfection through human direct or indirect intervention. The present invention also encompasses any cell which has been genetically engineered so as to produce at least one polypeptide according to the invention.

The present invention also encompasses any cell that has been deleted of a mutant polynucleotide according to the invention.

When a polynucleotide of the invention further comprises a sequence coding for a nuclearization signal, or is coupled to a nuclearization compound, it is very useful as drug vector so as to bring a drug into contact of the nucleus of a cell. For example, polynucleotides such as SEQ ID N°1, SEQ ID N°4 are capable of autonuclearization. The present invention thus encompasses any drug nuclearization vector which comprises at least one polynucleotide of the invention, or at least one polynucleotide coding for a complex of the invention, these polynucleotides either comprising a sequence coding for a nuclearization signal or being coupled to a nuclearization compound. Examples of appropiate drugs include those which are intended for treatment of the nucleus of a cell, e.g. for inhibiting mitosis, such as the p53 antioncogene.

The present invention provides with pharmaceutical compositions, and in particular with compositions for therapy and/or palliation and/ or prevention of an undesired state, *i.e.* with drugs.

According to an aspect of the invention, there is thus provided with pharmaceutical compositions which comprise, in a quantity effective for the desired effect, at least one polypeptide of the invention, and/or at least one product of stimulation activity according to the inventio (see the above "binding products" section), and/or at least one polynucleotide of the invention, and/or at least one complex of the invention, and/or at least one polynucleotide coding for a complex of the invention, and/or at least one transfection vector coding for a complex of the invention, and/or at least one transfected cell coding for a complex of the invention. Such a composition may also comprise alternatively comprise and/or at least one stabilization agent coding for a complex of the invention. Such pharmaceutical compositions of the invention are notably useful for inhibiting the proliferation of a cell population, and/or for stimulating the differentiation of a cell population, and/or for stimulating the establishment of a quiescent state in a cell population. The present invention particularly encompasses antitumoral compositions comprising in a quantity effective for the desired effect, at least one polypeptide of the invention, and/or or at least one polynucleotide of the invention, and/or at least one complex of the invention, and/or at least one polynucleotide coding for a complex of the invention, and/or at least one stabilization agent coding for a complex of the invention, and/or at least one transfection vector coding for a complex of the invention, and/or at least one transfected cell coding for a complex of the invention. Such an antitumoral composition may *e.g.* be intended for the prevention and/or palliation and/or treatment of tumors such as neuroblastoma, retinoblastoma, pheochromocytoma, breast tumor, kidney tumor, colon tumor, vesica tumor, prostate tumor.

According to another aspect of the invention, there is also provided with pharmaceutical compositions which comprise, in a quantity effective for the desired effect, at least one binding compound (*e.g.* purified serum, antiboby, and/or monoclonal antiboby of the invention) of inhibition activity as above-defined for the present invention, and/or at least one of antisens polynucleotide of the invention, and/or at least one mutant polypeptide or polynucleotide of the invention, and/or at least one transfection vector comprising an antisens polynucleotide of the invention, and/or at leat one cell transfected by such a vector or such a polynucleotide. Such compositions are notably useful for stimulating the proliferation of a cell population, and/or for inhibiting the differentiation of a cell population, and/or for inhibiting the establishment of a quiescent state in a cell population. The present invention thus encompasses compositions for cell regeneration, and for example for neuronal cell regeneration which comprises at least one binding compound (*e.g.* purified serum, antiboby, and/or monoclonal antiboby of the invention) of inhibition activity as above-defined for the present invention, and/or at least one of antisens polynucleotide of the invention, and/or at least mutant polypeptide or polynucleotide of the invention.

The pharmaceutical compositions of the invention may further comprise any compound useful for its efficacity and efficiency, they can e.g. comprises coagents, and/or any physiological vehicle. The dosage form can be chosen and ajusted by the skilled persons according to the effect that is desired, and to the administration route chosen. The pharmaceutical composition may thus have any appropriate form, this includes tablet, patch, solution, spray, injectable solutions, powder, colloidal solutions, paste. Posology can be determined and adjusted by the skilled persons according to the patient state, age, weight, sex.

The present invention also encompasses any reagent or kit which comprise at least one product chosen among the group consisting of the polypeptides according to the invention, the binding products according to the invention, the polynucleotides according to the invention, the complexes according to the invention the transfection vectors of the invention, and the cells of the invention.

Such reagent or kit may also comprise buffer and/or conservative solutions, and/or may comprise additional reagents.

According to another aspect of the invention, a method for diagnosing an undesired state for a human person or an animal, or for following the evolution of this undesired state, namely along the course of a medical treatment. This method of the invention comprises

- the observation in a biological sample of polypeptides according to the invention at a level significantly inferior to the normal level in the nuclear part of said biological sample, and/or
- the observation in a biological sample of polypeptides of the invention at a level significantly higher in the cytoplasmic part of this biological sample compared to the level observed in the nuclear part of the same sample.

It may also, or alternatively comprises :

- the observation of the presence in said sample of a mutant polynucleotide or of a mutant polypeptide according to the invention, namely at a level significantly higher than the normal level.

Such observations can be made by any common polypeptide/polynucleotide detection, or quantification procedure that the skilled persons find appropriate; examples of such procedures are further given in the below examples. Examples of a appropriate reactants for observation of the polypeptides of the invention notably comprise at least one binding compound of the invention as above-defined (serum, antibody, monoclonal antibodies). Appropriate samples include cells, histological sections, and cell extracts such as nuclear extracts, cytoplasmic extracts, polypeptidic extracts, polynucleotidic extracts, biopsies.

This method of the invention is useful for undesired state wherein cell hyperproliferation is involved, namely for cancerous state, such as neuroblastoma, retinoblastoma, breast, colon, kidney, vesica, prostate, tumors.

The present invention also relates to a method for identifying an anti-mitotic agent, characterized in that:

- a test compound is placed in contact with a cell which expresses at least one polypeptide according to the invention in a compartment different from the nucleus, such as the cytoplasm,
- the test compound is identified as an anti-mitotic agent when it can be observed that said at least one polypeptide has moved towards or into the cell nucleus.

Such an anti-mitotic agent may *e.g.* act one the phosphorylation level of said at least one polypeptide.

Said method may correspond to a screening method wherein said test compound is chosen among a chemical and/or biological library.

Said cell may be any cell expressing said polypeptide in the appropriate compartment, namely in the cytoplasm. It may *e.g.* be a human cell, an animal cell, a cell from a cell line, a tumoral cell. The test compound is preferably placed into contact with such a cell under conditions of the physiological type, such as *in vitro* conditions mimiking the *in vivo* ones (*cf*. above).

Other and further characteristics, advantages and variant embodiments of the invention can be read by the skilled person in the below-given examples. These examples are given for illustration purposes, they do not limit the scope of the invention.

In these examples, reference is made to figures 1 to 9 which represent:

- Figures 1A and 1B illustrate PATF nucleic (SEQ ID N°1) and amino acid (SEQ ID N°2, 3, 5) sequences :
Figure 1A illustrates the nucleic and deduced amino acid sequences of the quail PATF cDNA. The unbold part of the sequence (position 429-1082 on SEQ ID N°1; this 429-1082 ADNc sequence corresponding to SEQ ID N°6, 654 bp) corresponds to the inserted fragment in the RCAS vector for the antisense strategy and includes the bold ATG initiator.
Figure 1B illustrates the amino acid sequence (SEQ ID N°2, 3, 5; 277aa) deduced from the quail PATF nucleic sequence (SEQ ID N°1, 2196 bp) corresponding to the open reading frame (SEQ ID N°4, 834 bp). We note the leucine zipper domain (green) at the N-terminus part and the basic domain (red) containing a nuclear localization signal at the C-terminus part of the protein.

- Figures 2A, 2B and 2C illustrate the expression of PATF mRNA. Figure 2A illustrates the northern blot analysis of PATF RNA expression during chick neuroretina development. Total RNAs (15µg per lane) prepared from retina at the indicated stages were loaded. Transcripts PATF are developmentally regulated and the signal is more intensely expressed at ED17 on when the bulk of neuroretina cells is postmitotic. Only one transcript of 6.8-7 kb is detected. Figures 2B and 2C illustrate the RT-PCR products corresponding to a 255 bp fragment (SEQ ID N°19= from position 724 to position 978 on SEQ ID N°1) of the coding region were probed with an internal primer (see Example 1, Material and Methods). On Figure 2B: chick tissues, heart (1), brain (2), liver (3), fibroblast (4) and adult retina (5). On Figure 2C: mammalian tissues, mouse retina (1), rabbit retina (2), mouse brain (3), mouse spleen (4), mouse thymus (5), mouse cerebellum (6), mouse central nervous system ED15 (7), mouse liver (8).

- Figures 3A and 3B illustrate the expression of PATF protein during the chick development.
On Figure 3A, protein cell lysates (50 µg per lane) from quail retina at the indicated stages were immunoblotted with anti-PATF antibody (I) and preimmune serum (PI). PATF signal is already detected at ED5 and remained at a low level during the proliferation stages (ED5, ED8, ED10, and ED11). PATF signal becomes more intense when retinas are post mitotic (ED12, ED17, and 7 days post-hatching). No significant signal was detected with the preimmune serum (PI).
On Figure 3B, anti-PATF was used to probe protein from nuclear extracts in retina tissues at ED5 and ED17. As expected the amount of the nuclear PATF protein is highly expressed at ED17 compared to ED5.

- Figures 4A and 4B illustrate the immunolocalization of PATF in chick neuroretina.
Figure 4A illustrates the confocal microscopy observations of immunofluorescence for PATF (green) combined with propidium iodide as a nuclear stain (red) shown on cryostat retina sections (10 µm ) during chick development at the indicated stages. A nuclear localization of PATF is visualized by the yellow staining. PE: pigmented epithelium, ONL: outer nuclear layer, OPL: outer plexiform layer, INL: inner nuclear plexiform layer, GCL: ganglion cell layer.
On Figure 4B, retina cell suspensions were prepared from ED5, ED8, ED17. PATF localization was performed by immunocytofluorescence. Note that the PATF staining becomes progressively nuclear during the development. The majority of cells show a nuclear staining (yellow) at ED17 stage when cells are quiescent.

- Figure 5 illustrates the immunocytochemistry of PATF (on the right) in various cell lines compared to control (on the left) : PATF is expressed at a low level in human neuroblastoma cell line (SH-SY5Y), rat pheochromocytoma cell line (PC12) and human retinoblastoma cell line (Y79). Note that the labeling of PATF is only cytoplasmic, this indicates that the fact that these cell lines are in active proliferation.

- Figures 6A and 6B illustrate the effect of a decrease in the amount of PATF by an antisense strategy.
Figure 6A illustrates *in vitro* results: immunostaining, probed with anti-p27gag, anti-PATF, anti-BrdU antibodies was realized on non infected cells (N I), infected with sense (S) and antisense (AS) RCAS constructs. The amount of virus infection detected by anti-p27gag is similar in both sense or antisense RCAS constructs (*a*). A significant decrease of the amount of PATF is observed when cultures were infected with the antisense constructs (*c*). No significant staining was observed with the preimmune anti-PATF serum (PI ) (*b*). With antiBrdU the S phase staining is observed in numerous foci of cells only when cultures cells were infected with the antisens RCAS construct (*d*)
Figure 6B illustrates *in vivo* results: retinas cryosections (15µm thick) of injected eyes at ED 4.5 with: sense RCAS construct as control (*a*) ; and antisense RCAS construct (*b*, *c, d*). Morphological alterations, particularly the presence of numerous rosettes (*b* and *c*, asterisks) and retinal infoldings (*c* and *d*, arrows) were observed in injected antisense RCAS retina. Scale bar, 50 µm.

- Figures 7A and 7B illustrate PATF antibody supershift assays in chick retina. The labeled probes include C/EBP, AP-1 and E2F binding sites identified at the bottom. On Figure 7A, the gel shift assay with the indicated binding sites was performed with 20 ng purified PATF protein alone (lanes 1, 4, 7), plus 200 ng of respective competitor (lanes 2, 5, 8 ) and plus the anti-PATF antibody diluted to 1:200 (lanes 3, 6, 9) in 15 µl reaction mixture.
On Figure 7B, the gel shift assays were performed as described above with 5 µg nuclear extract from retina tissue at ED5 and ED17 without competitor (lanes 1, 4), with respective competitor (lanes 2, 5) and with the anti-PATF antibody (lanes 3, 6). Control (lane 7) was performed with 5 µg ED17 nuclear extract in the presence of E2F probe incubated with the preimmune serum. Shifts were observed with E2F (large arrow ) and C/EBP probes (small arrow) We observe a clear supershift with anti-PATF only when E2F sequence was used (asterisk). This supershift is obtained either with purified protein (A) and with ED5 and ED17 protein extracts (B).

- Figures 8A and 8B illustrate protein interactions between PATF and E2F family members.
Figure 8A illustrates the immunoprecipitations analysis performed with ED17 retinas protein extracts. Lysates were immunoprecipitated with preimmune serum (PI), anti-PATF, anti-E2F1 and anti-E2F4 antibodies. Complexes were then probed with anti-PATF antibody. E2F4 coimmunoprecipitates with PATF whereas the association between E2F1 and PATF is not significant.
Figure 8B illustrates the immunohistochemistry of E2F4 during the development (control on the left). At ED8 (middle) we observed an E2F4 cytoplasmic localization while at ED17 (right), E2F4 shows a nuclear localization similarly to that of PATF.

- Figures 9A, 9B and 9C illustrate functional interactions of PATF (left) with E2F4 (middle) and E2F1 (right).
Figure 9A illustrates the PATF, E2F4, E2F1 protein expressions in retina at two development stages: ED5 (proliferating tissue) and ED17 (quiescent tissue). Note a strong increase of PATF and E2F4 expressions in ED17 compared to ED5. In contrast, E2F1 is only detected at ED5 and no significant signal was seen at ED17.
Figure 9B illustrates the PATF, E2F4 and E2F1 protein expressions in RCAS infected cells (primary cultures). Infected cells with the RCAS vector alone as control (C), infected cells with the RCAS vector containing the PATF sequence in antisense orientation (AS). In antisense infected cells, we observed a decrease of PATF expression as well as E2F4 protein. Whereas in the same extracts, E2F1 level is significantly increased.
Figure 9C illustrates the RT-PCR of two target genes activated by E2F1 in primary cultures infected cells. RNA samples were prepared from cultures infected with control RCAS vector (C) or with primary cultures infected with the RCAS PATF antisense vector (AS) . Specific primers for cDNA amplification of the dihydrofolate reductase (DHFR) and DNA polymerase $\alpha$ (DNA POL $\alpha$) were used as described in Material and Methods. As expected, both genes are upregulated in antisense infected cells.
« ED » is herein meant as Embryogenesis Day; « transcription factor » as any molecule that can under appropriate conditions control a process of DNA transcription.
Many of these figures are better read when shown in colors. Accordingly, color-printed figures have been filed as the original drawing set at the receiving patent office.

**<u>Exemple 1</u> : Isolation of PATF (aa sequence : SEQ ID N°2, N°3, N°5 ; polynucleotidic sequence SEQ ID N°1, N°4)**

**Materials and Methods.**

**Northern blot analysis**

Total RNAs was isolated by the guanidium/thiocyanate method (Chirgwin et al., 1979, Isolation of biologically active ribonucleic acid from sources enriched in ribonuclease. *Biochemistry* 18: 5294-5299). 15µg of total RNAs was subjected to electrophoresis in a 1% agarose gel containing 6% formaldehyde in MOPS buffer. The relative amounts of mRNA were checked by ethidium bromide staining and comparison of ribosomal RNA levels. The RNA was transferred to a nylon membrane and probed for 20h at 42°C in standard conditions (Maniatis et al., 1982 Molecular cloning: a laboratory manuel. NY Cold Spring Harbor Laboratory). cDNA probes were prepared by random primer synthesis (Amersham kit). The membrane was placed against X-ray film at -80C for 48h with an intensifying screen.

**RT-PCR analysis**

**PATF mRNA expression in chick and mouse tissue:** A random-primed single strand cDNA was synthesized, from total RNA extracted from various tissues by reverse transcriptase and was amplified using the oligonucleotide primers, 5'-TCCTATGCTTGACTATTAGC-3' (SEQ ID N°7) and 5'-TACAGCTGCTGGGAGCTGG-3' (SEQ ID N°8). For each amplification, we obtained a PCR product with the expected size of 255 bp (SEQ ID N°19). Primers were synthesized by GENSET. Amplification was performed over 40 cycles in a Perkin-Elmer/Cetus DNA thermal cycler using a cycle of denaturation for 1 min at 94°C, annealing for 1 min at 56°C and extension at 72°C for 1 min. PCR products were separated electrophoretically in a 1.3% agarose gel and transferred to nylon membrane using the alkaline transfer method (Chomczynski et al., 1984. Alkaline tranfer of DNA to plastic membrane.

*Biochem. Biophys. Res. Commun.* 122: 340-344). The $^{32}P$-labeled oligonucleotide used as a probe for the PCR product was: 5'-CCTGACATCCCTACATCACCGCAT-3' (SEQ ID N°9).

**DHFR and DNA POL α expressions in primary culture cells:** RT-PCR method was performed as previously described by Vigo et al. 1999. CDC25A phosphatase is a target of E2F and is required for efficient E2F-induced S phase. *Mol. Cell Biol.* 19: 6379-6395). Primers used for the cDNA amplification for DHFR, 50°C, 30 cycles using 5'-TGGTTCTCCATTCCTGAG-3' (SEQ ID N°10) and 5'-AGGACGTACTAGGAACAG-3' (SEQ ID N°11), for DNA POL α, 50°C, 30 cycles, using 5'-AGCTGATGGATGGTGAAG-3' (SEQ ID N°12) and 5'-AATACTCCTCTGCTGAGG-3' (SEQ ID N°13), for GAPDH, 54°C, 22 cycles using 5'-ATCCGTTGTGGATCTGACATGC-3' (SEQ ID N°14) and 5'-TGTCATTGAGAGCAATGCCAGC-3' (SEQ ID N°15).

**RCAS vector constructions and transfection.**

The RCAS vector is an helper -independent retroviral vector derived from the Rous Sarcoma virus (RSV) (Hughes et al., 1987. Adaptor plasmids simplify the insertion of foreign DNA into helper-independent retroviral vector. *J. Virol.* 61: 3004-3012). In this vector, the region encoding the oncogene src ,has been excised by manipulation of a cloned DNA copy of the viral genome and replaced by a synthetic DNA linker containing the recognition site for the restriction enzyme: ClaI. We inserted at the unique ClaI site, a 654 bp fragment of the PATF sequence flanked by the two ClaI sites. This fragment contains the ATG initiator and corresponds to the sequence from 429 bp to 1082 bp (Fig. 1A). Sequence orientation was determined by sequencing. RCAS cloned retroviral DNA (7μg) was transfected into chick embryos fibroblasts ($2x10^5$ cells/$cm^2$ in $25cm^2$ flask) by overnight calcium phosphate precipitation followed by two washes with phosphate buffer saline (PBS) (Chen and Okayama, 1987. High efficiency transformation of mammalian cells. *Mol. Cell Biol.* 7: 2745-2752). When cells became confluent, they were expanded in $150cm^2$ flask (1/12). A part of cells were fixed to check the presence of virus by immunochemistry with an antibody against a protein of the viral capsid (rabbit anti-p27 IgG fraction (p27gag), Life Sciences Inc.). When they were nearly confluent, the medium was removed and replaced with minimum volume of fresh medium. The supernatant was harvested 3 hours later, filtered on 0.45μm filter (Nalgen) to remove cellular debris and ultracentrifugated at 100 000 g for 1 hour. The pellet was resuspended in 1/100 of volume in culture medium, aliquoted and frozen at -80°C. The concentrated virus was used to infect primary cultures of ED 5 neuroretina cells, or to inject into the chicken eyes at ED 4.5. Cultures were analysed 10 days after infection with the anti-p27 gag antibody (1/1000 in PBS 0.1% TritonX100) to show the presence of the virus and with the anti-PATF (1:100 in PBS 0.1% Triton X100) and the mouse monoclonal anti-BrdU (FITC Becton Dickinson 1/10 in PBS, 0.5% Tween 20). The eyes injected were also analysed 10 days after injection for histology.

**PATF antibody:**

PATF serum was prepared against three specific peptides, chosen between the leucine Zipper domain and the basic domain of the protein.

**Immunohistochemistry**

*In vivo*

Eyes were fixed overnight with 4% paraformaldehyde in 0.1M PBS, pH 7.4. After cryoprotection by immersion in 20% sucrose, 10μm thick cryostat sagittal sections were collected on "siliconated" slides and kept at -20°C. For immunolabeling the retina sections were permeabilized in blocking solution (PBS skim milk 5%, Triton X100 0.1%) for 45min. Sections were incubated 1 hour with the anti-PATF serum diluted 1/200 in PBS skim milk 5%, Triton X100 0.1%) and then with a 1/50 dilution of F(ab')2 FITC goat anti-rabbit serum (CALTAG code n° L43001) in PBS skim milk 5%, Triton X100 0.1% for 1 hour. Sections were stained with propidium iodide (0.4 mg/ml in PBS). Control was performed using standard rabbit IgG.

*In vitro*

**Chick neuroretina primary cultures.**

Chicken neuroretinas were explanted at ED6 and incubated with 0.25% of trypsin in Versen during 30 minutes at 37°C. After gentle dissociation, cells were cultured during ten days in Eagle's basal medium with 10% fetal bovine serum and infected with the RCAS sense or antisense virus. Controls were uninfected cells. Cultures were incubated at 37°C and medium renewed twice a week (Crisanti-Combes et al., 1982; Expression of neuronal markers in chicken quail and neuroretinal cultures infected with Rous sarcoma virus. *Cell Diff.* 11: 45-54). Crisanti et al., 1985. Glutamic acid decarboxylase activity is stimulated in quail retina neuronal cells transformed by Rous sarcoma virus and is regulated by pp60v-src. *EMBO J*. 4: 1467-1470). Immunohistochemistry was performed as described above but, cellular cultures, were fixed only 30min in 4% paraformaldehyde in 0.1M PBS, pH 7.4.

**Cell lines culture.**

Human Y79 retinoblastoma cells, obtained from ECACC (Porton Down, Salisbury, UK) were cultured as a suspension in RPMI 1640 medium (Reid et al., 1974. Characteristics of an estabished cell line of retinoblastoma. *J. Natl Cancer Inst.* 53: 347-360). The SH-SY5Y cells, a human neuroblastoma cell line (Biedler et al., 1973. Morphology and growth, tumorigenicity and cytogenetics of human neuroblastoma cells in continuous culture. *Cancer Res.* 33: 2643-2652) were cultured in Dulbelcco's modified Eagle's medium. Rat PC12 cells were maintained in Dulbelcco's modified Eagle's medium (DMEM) supplemented with 10% fetal calf serum, 5% horse serum. The three cell lines were cultured at 37°C with penicillin (100 IU/ml) and streptomycin (50µg/ml).

**Confocal microscopy analysis.**

Retinas were treated with trypsin on ED8, ED15 and PN7. Cells were collected by centrifugation and fixed with 4% paraformaldehyde (pH 7.4) for 1 hour. For confocal microscopy, ED8, ED15 and PN7 retina cells were first incubated with the anti-PATF antibody (dilution at 1:200) for 30 min, then with secondary antibody, goat anti-rabbit F $(ab')_2$ fragment conjugate FITC (diluted at 1:50, CALTAG) in PBS for 30 min, then counterstained with 20 µg/ml of Propidium iodide. Cells were mounted on glass slides with Mowiol. Confocal microscopy and scanning were performed using an ACAS 570 Interactive laser Cytometer (Meridian Instruments, Inc, Okemos, MI) equipped with confocal optics. The system consisted of a 5W argon ion laser, an Olympus IMT-2 microscope with a 100X oil immersion objective, Z axis control, an XY scanning stage, and a variable spindle aperture, under 80486 computer control.

**Immunoprecipitation and Western blotting.**

Retina tissues were homogenized in lysis buffer (50 mM Tris-HCl, pH 7.5, 2mM EDTA, 150mM NaCl, 1mM phenylmethylsulfonyl fluoride, 0.5% NP40 and Boehringer Mannheim protease inhibitor cocktail). After 30 min at 4°C, homogenates were cleared by centrifugation at 10.000g for 15 min. The supernatants were immediately assayed for immunoblotting and immunoprecipitation experiments. For immunoblotting analysis, protein lysates (50µg per lane) were separated by SDS-polyacrylamide gel (10%) and transferred to nitrocellulose membranes and recognized by specific antibodies. The antibodies were detected using anti-rabbit, anti-goat or anti-mouse F(ab')2 fragment IgG peroxidase-linked. The immunoreactivity was visualized by enhanced chemiluminescence (ECL) detection system (Amersham Pharmacia Biotech). For immunoprecipitation analysis, 150-200 µg of protein extracts were treated with 5 µl of the appropriate primary antibody in volume of 100µl at 4°C for one hour. 3 mg of protein A sepharose in 100µl volume was then added and incubated for additional 1 hour. The beads were washed five times with 500µl of the same buffer, boiled in 40µl of SDS simple buffer and separated on 10% polyacrylamide gels. After semi dry transfert the blots were probed using appropriate antibody.
Antibodies were purchased from Santa Cruz Biotechnology, Santa Cruz CA: mouse monoclonal E2F-1 (KH95-sc251), rabbit polyclonal E2F-4 (C20-sc866), rabbit polyclonal DPI (K20-sc610), rabbit polyclonal DP2 (C20-sc829). Peroxidase conjugated antibodies were from CALTAG. Protein concentrations were determined by the method of Bradford (1976).

**PATF protein purification.**

Anti-PATF antibody was covalently coupled to NHS-activated Sepharose High Performance column as manufacturer's recommendations (Pharmacia). Columns were then used to purify PATF from ED17 retinal protein extracts according to Pharmacia protocol. Each fraction eluated was then tested by western blot to identify the PATF fraction.

**Electromobility shift assays (EMSAs)**

EMSAs were performed as described previously (Rouet et al., 1992). A potent enhancer made of clustered liver-specific elements in the transcription control sequences of human α1-microglobulin/bikunin gene. *J. Biol. Chem.* 267: 20765-20773) with ED5 and ED17 retinal nuclear protein extracts and PATF purified protein. Three oligonucleotides, C/EBP:5'-CTAGGATATTGCGCAATATGC-3' (SEQ ID N°16), AP-1: 5'-TTTCTGAAGTCAGTCAGCC-3' (SEQ ID N°17), E2F: 5'-TAGTTTTCGCGCTTAAATTTGA-3' (SEQ ID N°18) and their respective complementary sequences were annealed and radiolabeled using polynucleotide kinase and gamma-$^{32}$P-ATP. Binding reactions were performed in 15 µl of a reaction mixture containing 10 mM Hepes buffer, pH 7.9, 30 mM KCl, 1 mM $MgCl_2$, 9mM spermidine, 0.5 mM DTT, 10% glycerol, 1.5 µg of poly (dI-dC), and 1 µg of sonicated salmon sperm DNA. Five µg of nuclear protein extract or 20ng of PATF purified protein were preincubated in this mixture for 5 min at 4°C. Then 1ng of kinase-labeled double-stranded oligonucleotide as a probe and competitor were incubated for 15 min on ice. The DNA-protein complexes were loaded onto a prerun 5% polyacrylamide gel (29:1) in 0.25 x TBE, dried and electrophoresed at 12 V/cm. The double-stranded oligonucleotides used as competitor were obtained by annealing two complementary oligomers. Probe supershifts were performed by incubation for 30 min of the reaction mixture with 1µl of the anti-PATF antibody (final dilution 1:200) at 4°C.

**Results**

An autosubtracted cDNA library has been constructed (Bidou et al., 1993. A novel cDNA corresponding to transcripts expressed in retina post-mitotic neurons. *Mech. Dev.* 43: 159-173), and one of its clones is herein characterized: the PATF cDNA.

**Sequence analysis of PATF cDNA.**

A library prepared from total mRNA from neuroretinas of two days postnatal quail was screened with the PATF cDNA, which had been isolated from the subtracted library. We isolated 3 overlapping cDNAs. The sequence obtained was 2196 bp long (SEQ ID N°1) and contained the full-length 834 bp open reading frame (SEQ ID N°4) (*cf.* Fig. 1A). Primary structure analysis and comparison with database sequences indicated that the PATF nucleotide sequence had no significant similarity to listed sequences. The deduced amino acid sequence corresponds to a protein of 277 amino acids (Fig. 1B SEQ ID N°2, N°3, N°5). The PATF protein contains a leucine zipper motif, a basic domain and a nuclear localization sequence (NLS). The leucine zipper consensus sequence is at the N-terminus and the basic domain is localized at the C-terminus. Seven putative protein kinase C phosphorylation sites have been identified at positions 16, 74, 179, 192, 203, 269, 275 on SEQ ID N°2, 3, 5.

**PATF mRNA expression.**

To determine the pattern of PATF mRNA levels during retinal development, four stages, ED5, ED8, ED15 and 7 days post-hatching were tested for PATF transcripts expression by northern blot analysis in chick neuroretina (Fig. 2A). Only one transcript of 6.8 -7 kb was detectable . The amount of PATF mRNA increased by ED8, ED15 and 7 days post-hatching, in parallel to the establishment of the quiescent state .
We investigated whether PATF mRNA was also present in other tissues, by RT-PCR in various chicken (Fig. 2B) and mammalian tissues (Fig. 2C). A 255 bp fragment (SEQ ID N°19) was amplified with the same primers (see experimental procedures), in chicken tissues including heart, brain, liver and fibroblasts (Figure 2B). In mammalian tissues, PATF mRNA was present in rabbit and mouse retinas, and also in other mouse tissues including brain, spleen, thymus, cerebellar and liver. No PATF mRNA was detected in the mouse central nervous system at ED15 (Fig. 2C).

**Western blot analysis**

Western blot analysis was carried out (as described in Omri et al., 1998. Retinal dysplasia in mice lacking p56 lck. *Oncogene* 16: 2351-2356) with total protein extracts from retina at various stages of embryogenesis between ED8 and 7 days post-hatching. As shown in Fig. 3 A, with anti-PATF antibody (I) a clear signal was detected with an apparent molecular weight of 50kDa. A faint signal was present at ED5, ED8 and ED10. PATF protein expression increased at ED11, reaching its maximum at ED12, when cells become postmitotic, and remaining at this level for the rest of development. No significant specific immunoreactivity was detected in the control experiment performed with preimmune serum (PI). As expected, PATF protein was also detected in nuclear extracts (prepared according to Graves et al., 1986. Homologous recognition of a promoter domain common to the MSV LTR and the HSV tk gene. *Cell* 44: 565-576) and the PATF signal was stronger in quiescent neuroretina at ED15, than in proliferating retina at ED5 (Fig. 3B). Thus PATF expression closely parallels the establishment of the quiescent state, suggesting a major role for this new gene in the control of cell proliferation.

**Localization of PATF in chick neuroretina and various cell lines.**

**Localization in eye sections.**

We investigated the chronology and topography of PATF protein synthesis in chick neuroretina at various stages of embryogenesis, by studying serial eye-sections by immunocytochemistry, with the anti-PATF serum (Fig.4A). In early stages of development, (ED5, ED8), retinas were not yet differentiated, or organized into layers and most of the cells were in mitosis. At this stage, PATF staining was restricted to a few cells and the signal is essentially limited to the cytoplasm. At ED10, ED17 and 7 days post-hatching, the retina was definitively organized. The PATF became widespread in majority in all retinal layers and more faintly in the outer nuclear layer, which consists of photoreceptor cells. The signal become more intense and was restricted to the nucleus in many cells in all the various cell layers. It is interesting to note that PATF localization in mouse retina was similar to that described for chick retina (data not shown). The staining showed the same distribution within the equivalent cell layers.

**Localization in retina cell suspensions.**

Retinas were dissociated from eyes of chicks at the ED5, ED8, ED17. Cell suspensions were stained with propidium iodide and used for experiments to localize PATF by immunofluorescence (Fig.4B). At ED5, only several cells exhibited a staining with anti-PATF serum. At ED8, PATF localization was clearly detected in the cytoplasm or in the nucleus, or both in the cytoplasm and the nucleus . We also identified cells that did not express PATF. At ED17 and 7 days post-hatching (not shown), when cells are postmitotic, except a few cells, the major PATF staining was in the nucleus. Thus, PATF expression increases with the establisment of quiescent state and become predominantly nuclear at these stages.

**Localization in established cell lines.**

Parallely, we have performed immunocytochemistry study to determine whether PATF was expressed in PC12 rat pheochromocytoma cells, in SH-SY5Y human neuroblastoma cells and Y79 retinoblastoma cells (Fig.5). In our experimental conditions, these cell lines were in active proliferation. PATF, as expected, was detected exclusively in the cytoplasm compartment. No significant nuclear labeling was observed. This results points to the importance of the nuclear localization of PATF in the control of cell proliferation.

**Involvement of PATF, *in vitro* and *in vivo*, in the control of cell proliferation.**

We investigated whether PATF was involved in the control of cell proliferation using a retroviral antisense strategy to repress PATF protein expression. We have inserted a 654 bp fragment (sens orientation : SEQ ID N°6 ; antisens orientation : SEQ ID N°24) of the PATF sequence, containing the initiator ATG in antisense orientation, into the RCAS vector (Hughes et al., 1987. Adaptor plasmids simplify the insertion of foreign DNA into helper-independent retroviral vector. *J. Virol.* 61: 3004-3012) to produce antisense mRNA in a continuous manner. The same portion of the PATF sequence was inserted into the vector in sense orientation as a control. Chick embryo fibroblasts transfected with the RCAS vector containing the DNA in a sense or antisense orientation produced virus particles in the culture medium. This medium was injected into the eye of chick embryos at ED4 or used to infect primary cultures of neuroretina cells plated at ED6 when cells were undifferentiated and in proliferation.

***In vitro experiments.***

We checked the efficacity of viral infection after ten days, by testing some of the cells for the presence of the virus, by immunocytochemistry, using an antibody directed against the protein of the capsid p27 gag. Cultures prepared in parallel were used to assess p27gag and PATF expression. The proliferation status was determined with an antiBrdU antibody. Results are shown in Fig.6A The same strong signal was detected in cells infected with both sense or antisense RCAS constructions. No signal was detected in uninfected cells. The labeling with the anti-PATF antibody shows a significant decrease with cells infected with the antisense RCAS construct compared to the control or sense-infected cells. In parallel, in cultures, where PATF expression is decreased, we observed foci of cells stained with the antiBrdU. In non- infected cells or sense-infected cells we observed a similar staining with anti-PATF and we did not detect any cells in S phase. Thus decreasing PATF expression prevented cells from withdrawing from the cell cycle, since in these conditions, we clearly observed additional cells in S phase.

***In vivo experiments.***

Ten days after virus injection, we performed an histological study of eye cryosections. Eyes injected with the antisense RCAS construct showed retinal dysplasia. Five series of similar experiments were performed and the retinal abnormalities were found to be reproducible. The morphological changes observed consisted mostly of retinal detachment, retinal infolding and rosette formation and resulted in the loss of the normal architecture of the tissue (Fig.6B). Eyes injected with the sense RCAS construct had normal morphology. These results, obtained using an antisense strategy in vitro and in vivo, demonstrate that PATF plays a major role during development.

**PATF DNA binding activity .**

We investigated the DNA binding properties of PATF, using three oligonucleotides, corresponding to binding sites for different basic domains transcription factors. Two are involved in growth arrest and terminal differentiation: the CCAAT/ enhancer-binding protein (C/EBP$\alpha$) ( Osada et al., 1996. DNA binding specificity of the CCAAT/enhancer-binding protein transcription factor family. *J. Biol. Chem.* 271: 3891-3896) and E2F binding sites (Umek et al., 1991; Slansky and Farnham, 1996. Introduction to the E2F family : protein structure and gene regulation. *Curr. Top. Microbiol. Immunol.* 208: 1-30). The third oligonucleotide corresponds to a binding site from early response genes, involved in a diverse set of transcriptional regulatory processes: the activation protein-1, AP1 (Olive et al., 1997. A dominant negative to activation protein-1 (AP1) that abolishes DNA binding and inhibits oncogenesis. *J. Biol. Chem.* 272: 18586-18594). These binding sites were tested in electromobility shift assays (EMSAs) using an affinity-purified PATF protein fraction from ED17 retina or nuclear protein extracts from retina at ED5 and ED17. A supershifted complex containing purified PATF protein, was obtained only with the E2F probe (Fig.7A, lanes 7-9). No complex was detected with purified protein and the C/EBP or AP1 probes (Fig.7A, lanes 1-6). In experiments performed in parallel with ED5 and ED17 nuclear protein extracts (Fig.7B), no supershift was observed with the C/EBP probe (Fig.7B, lane 3). In contrast, a supershift was observed with anti-PATF when the E2F sequence was used (Fig.7B, lane 6) in control assay performed with preimmune serum no supershift was detected (Fig.7B, lane 7). The specificity is indicated by competition by E2F site with non labeled oligonucleotides (Fig. 7A, lane 8) (Fig. 7B, lane 2, 5). Our results clearly show that PATF binds to the E2F site oligonucleotide. Furthermore, the profiles of these complexes differ at ED5 and ED17 since with ED5 extract, a single band was detected whereas with ED17 extracts, two distinct bands were detected suggesting that PATF binds to DNA as homodimers or as heterodimers in association with a protein such as a member of E2F family .

**Physical interaction of PATF with the E2F family.**

We investigated the ability of PATF to interact with proteins involved in the cell-cycle machinery. We therefore performed experiments of coimmunoprecipitation with protein extract from ED17 retinas, using respectively standard rabbit immunoglobulin (PI), anti-PATF, anti-E2F1 and anti- E2F4. The immunocomplexes were then subjected to western blot analysis with anti-PATF antibody (Fig. 8 A). As expected, no significant signal was obtained for the immunocomplex precipitated with anti-E2F1 antibody, or with the control, whereas a clear signal was detected for the immunocomplex obtained with anti-E2F4 antibody. Moreover, immunohistochemistry study of E2F4 expression during the development (Fig. 8 B), shows that E2F4 localization is similar to that reported for PATF in Fig. 4 B. Indeed, E2F4 as PATF becomes nuclear in quiescent retina cells. Taken together, these results confirm the interaction between PATF and the members of the E2F family, and enabled us to determine more accuratly the nature of complexes, indeed we found that E2F4, but not E2F1 is associated with PATF.

**Functional interaction of PATF with E2F4 and E2F1**

We performed experiments to determine whether the physical interaction between PATF and E2F4 is functionally involved in the establishment of the quiescent state. We first compared in vivo the expression of PATF, E2F4 and E2F1 in retina at ED5 (growing cells) and ED17 (quiescent cells). We then studied, in primary cultures, using the RCAS antisense strategy the effect of the PATF protein decrease on the E2Fs expression and on two E2F1 target genes. Fig. 9 A shows protein expression of PATF, E2F4 and E2F1 in retina extracts at ED5 and ED17. At ED5, PATF and E2F4 were expressed as well as E2F1. At ED17 the expression of both PATF and E2F4 was strongly increased, while at this stage, no E2F1 signal was detected. Fig.9 B shows PATF, E2F4 and E2F1 protein expressions in primary cultures. We have previously shown that with an anti PATF or anti BrdU immunostaining (Fig.6A), PATF antisense strategy leads to a decrease of PATF expression and to additional mitosis. Using the same strategy, we observed that both PATF and E2F4 expression were decreased in the antisense-infected cells compared to the control. In contrast E2F1 expression was increased in the antisense infected cells extracts. To ascertain the effect of PATF decrease on E2F1 expression is followed by activation of E2F1 target genes, we studied the expression of two genes, namely dihydrofolate reductase (DHFR) and DNA polymerase α (DNA POLα), by RT-PCR (Vigo et al., 1999. CDC25A phosphatase is a target of E2F and is required for efficient E2F-induced S phase. *Mol. Cell Biol.* 19: 6379-6395). As shown in Fig.9 C, in antisense infected cells where PATF is downexpressed, we observed, as expected, a significant increase of DHFR and DNA POLα RNAs expressions compared to the control.
Taken together, these results suggest that PATF, by its association with E2F4, could act as a regulator of the quiescent state through the E2F pathway.

**Discussion**

We have isolated and sequenced a novel transcription factor, PATF, involved in the negative control of cell proliferation. This new gene was isolated using a subtractive approach between quiescent and active proliferating retinal cell clone. PATF presents characteristics of a transcription factor with a leucine zipper motif, a basic domain and a nuclear localization sequence. It also comprises phosphorylation sites. Our study shows that PATF expression and localization are developmentally regulated: 1°) PATF expression in retina, increases progressively during the retinal development parallely to the differentiation and the establishment of the quiescent state, 2°) the subcellular localization is closely depending on cell quiescent or proliferating state. In fact, PATF nuclear localization clearly correlates with proliferation arrest. To determine the implication of PATF in neuronal growth arrest we have performed experiments using RCAS retroviral antisense strategy. *In vitro*, the decrease of PATF protein expression after viral infection with PATF antisense construction prevents from withdrawing from the cell cycle and since it causes additional mitosis in retinal primary cultures and that is consistent with PATF implication in neuronal growth arrest. *In vivo*, the decrease of PATF expression by antisense retroviral construction affects the neuroretina development, leading to morphological alterations, such as dysplasia.
Since PATF presents characteristics of a transcription factor we performed gel shift assays to determine whether PATF binds to DNA. We have tested specific oligonucleotides of different DNA binding sites such as: C/EBP, E2F, AP1. Gel retardation assays were carried out with purified PATF protein or retina nuclear protein extracts from proliferation and quiescent stages of the development. With anti-PATF antibody our results show a clear supershift with the E2F site. No supershift was observed with C/EBP or AP1 sites. Thus PATF interacts with E2F binding site. Actually it is well established that the cellular genes activated by E2F encode proteins playing a critical role in the control of cell cycle. What then is the relation between PATF and E2Fs in the context of growth control. The known E2F species can be grouped into two subfamilies based upon certain structural and functional characteristics. E2F1 and E2F4 are considered as the two representant of these two subfamilies. In this context, it has been reported that E2F1 is able to activate transcription following mitogenic stimuli (Slansky et al., 1993. A protein synthesis-dependent increase in E2F-1 mRNA correlates with growth regulation of the dihydrofolate reductase promoter. *Mol. Cell Biol.* 13: 1610-1618 ; Johnson et al., 1993. Expression of transcription factor E2F-1 induces quiescent cells to enter S phase. *Nature* 365: 349-352; Johnson, D. G., K. Ohtani, and J. R. Nevins. 1994. Autoregulatory control of E2F1 expression in response to positive and negative regulators of cell cycle progression. *Genes Dev.* 8: 1514-1525 ; Ohtani et al., 1995. Regulation of the cyclin E gene by transcription factor E2F. *Proc. Natl Acad. Sci. USA.* 92: 12146-12150) and that the downregulation of E2F1 gene expression is necessary to the neuronal terminal differentiation in avian neuroretina (Espanel et al., 1998. Regulation of E2F-1 gene expression in avian cells. *Oncogene* 17: 585-594). n the other hand, the authors show that the p105Rb/E2F1 complex disappears when cells stop dividing, while the p130/E2F4 complex accumulates in quiescent neuroretina cells. Our results show a physical association between PATF and E2F4. This result correlates with the fact that E2F4 is expressed in quiescent state in contrast to E2F-1, -2 -3 and is in agreement with the authors reports. During the retina development, E2F1 is down regulated while E2F4 expression increases and becomes nuclear as PATF. Moreover using RCAS antisense strategy which blocks PATF expression, E2F4 level falls. In contrast, E2F1 level rises and is followed by transcription activation of two E2F1 target genes, DHFR and DNA POL α known to activate cell proliferation. Furthermore, as both PATF and E2F4 expressions are concentrated in the nucleus during the establishment of the retinal quiescent state, we suggest that this new transcription factor plays a critical role in the E2F cascade regulating cell cycle progression. The fact that PATF is excluded from the nucleus in various tumor cell lines, as it was described for E2F4 (Lindeman et al., 1997. The subcellular localization of E2F-4 is cell-cycle dependent. *Proc. Natl. Acad. Sci.* USA 94: 5095-5100), suggests a synergistic action of PATF and E2F4. PATF/E2F4 may thus act as an inhibitory complex bound to E2F sites in quiescent cells to prevent the activation of the E2F1 gene expression. The PATF/E2F4 complex appears as a new molecular signal involved in the activation of target genes responsible of the withdrawal cell cycle at the onset of the neuronal differentiation.

**Example 2: Production of anti-PATF purified sera**

Peptidic sequences specific of PATF have been searched on SEQ ID N°2, N°3, N°5 ; and those with the best immunogenic index have been selected, and produced at the immunograde level.

Such peptidic sequences namely comprise :

- Peptide 10-VT : .....VTLSAQGRGT (SEQ ID N°21)
- Peptide 10 YQ : .....YGPLTNPKPQ (SEQ ID N°22)
- Peptide 10-RR : .....RTLLLPAVSR (SEQ ID N°23)

These peptides are new : no homology has been detected on any awailable data bank.
These peptides have been injected into rabbits (N.Z. W, E.S.D. 75 ; female ; 2.3 kg at day O) according to standard immunisation procedures, such as :

| Day | Procedure steps |
|---|---|
| Day 0 | Collection of control serum (20ml) First intra-dermal injection (1ml/rabbit) 1 Tube for **2 rabbits** → 1ml Ag+ 1ml **COMPLETE FREUND** |
| Day 14 | Second intra-muscular injection (1ml/rabbit) 1 Tube for 2 rabbits → 1 ml Ag + 1ml **INCOMPLETE FREUND** |
| Day 28 | Third intra-muscular injection **(IDEM d14)** |
| Day 39 | Collection of test serum (5ml) and storing at +4°C (serum d39) |
| Day 49 | Fourth injection subcutaneous (1ml) 1 Tube for 2 rabbits →1ml Ag + 1ml **INCOMPLETE FREUND** |
| Day 60 | Collection of test serum (25-30ml) and storing at +4°C (serum d60) |
| Day 77 | Fifth injection intra-dermal (1ml/rabbit) **(IDEM d49)** |
| Day 88 | Collection of test serum (5ml) and storing +4°C (serum d88) |
| Day 99 | Collection of test serum (25ml) and storing at +4°C (serum d98) |
| Day 102 | Collection of test serum (25ml) and storing at +4°C (serum d102) |
| Day 104 | Collection of test serum (25ml) and storing at +4°C (serum d104) |
| Day 106 | Collection of test serum (25ml) and storing at +4°C (serum d106) |
| Day 109 | Total collection by total blood collection and storing at +4°C (serum d109) |

The reactivity of the sera obtained has been assayed for binding to peptidic sequences SEQ ID N°21, 22 and 23. From day 60, cell sera show a good reactivity with each of SEQ ID N°21, 22 and 23, with a prevalence for SEQ ID N°23.
The sera produced do bind to PATF (SEQ ID N°2, 3, 5), and remarquably do not bind to any known transcription factors, and/notably do not bind to any member of the E2F family.

**Example 3 : human PATF isolation**

Human cDNA Isolation:

Isolation of human sequence cDNA can be performed by screening of cDNA human library using full lenght open reading frame sequence of PATF.

Human protein purification:

Anti-PATF antibody was covalently coupled to NHS-activated Sepharose High Performance column as manufacturer's recommendations (Pharmacia). Columns were then used to purify PATF from human protein extracts according to Pharmacia protocol. After washing with 10 column volumes, each fraction eluated was tested by western blot to identify the fraction containing PATF protein.

**Example 4:**

Biopsies have been collected from kidney, colon, vesica, prostate, and breast human tumors. They have been assayed for presence and localization of PATF. Detection of PATF has been performed by immunocytochemistry using labelled sera of example 3.

PATF has been detected in all biopsies, and always localized in the cytoplasm.
Control observations on healthy tissues confirm PATF normal localization is in the nucleus.
PATF cytoplasmic localization thus correlates with a cellular proliferation.
It thus provides new means for the detection of cellular proliferation such as tumoral presence or development, which comprise the cellular localization of PATF by using e.g. an anti-PATF serum, and the observation of a totally or substantially nuclear localization (proliferation and/or differentiation is under inhibition) or of a totally or substantially cytoplasmic localization (proliferation and/or differentiation is under stimulation).

**Example 5:**

Biopsies from human tumors, such as breast tumors, show a cytoplasmic PATF localisation (see example 4), and this marks the abnormal (tumoral) status of these cells.
Such tumorals cells have been incubated in the presence of an anti-mitotic product of the proteine kinase C family such as TPA.
By comparison with control incubations, it is observed that the anti-mitotic product, besides from inhibiting tumoral proliferation, parellely induces the transfer of the initially cytoplasmic PATF into the nucleus.
This confirms that PATF nucleus localization correlates with inhibition of proliferation and/or differentiation, and with stimulation of the establishment of a quiescent state, even in treated tumoral cells.
The phosphorylation sites of PATF and its phosphorylation level are likely to be involved in such a transfer from cytoplasm to nucleus.
PATF, besides from representing a particularly useful nuclearization vector, thus represents a very useful diagnostic element for tumoral cases and very useful means for identifying new anti-mitotic compounds.

## Claims

**1.** Isolated polypeptide which is capable of inhibiting the proliferation of a cell population, and/or of stimulating the differentiation of a cell population, and/or of stimulating the establishment of a quiescent state in a cell population, **characterized in that** it:

- comprises at least one sequence of the leucine zipper domain type at the N-terminus,
- comprises at least one sequence of the basic domain type at the C-terminus, and
- comprises at least one sequence of the nuclearization signal type and/or is coupled to a compound capable of performing a nuclearization of said polypeptide into at least one cell of said population.

**2.** Isolated polypeptide according to claim 1, **characterized in that** said cell population comprises cells chosen among the group consisting of animal cells, vertebrate cells, bird cells, mammal cells, rodent cells, human cells, CNS cells, cells from heart, cells from brain, cells from cerebellum, cells from retina, nerve cells, neuronal cells, axonal cells, cells from a fibrous tissue, cells from a connective tissue, epithelial cells, fibroblats, cells from liver, from spleen, from thymus, and any cell which can be considered as a progeny cell or a descendent cell thereof.

**3.** Isolated polypeptide according to any one of the preceding claims, **characterized in that** said cell population comprises a cell population chosen among the group consisting of normal cells and hyperproliferative cells, such as tumoral cells.

**4.** Isolated polypeptide such as obtained by isolation from a neuroretina cell of a polypeptide of which apparent molecular weight is of about 50 kDa when measured by Western blot analysis

**5.** Isolated polypeptide of which sequence corresponds to a sequence chosen among the group consisting of SEQ ID N°2, SEQ ID N°3, SEQ ID N°5, and any polypeptidic sequence, namely any human polypeptidic sequences, such as obtained by conservative substitution and/or deletion and/or inversion of aminoacids of SEQ ID N°2, SEQ ID N°3, SEQ ID N°5.

**6.** Isolated peptides chosen among the group consisting of SEQ ID N°21, N°22, N°23.

**7.** Product which is capable of binding to a polypeptide according to any one of claims 1-6, such as a product chosen among the group consisting of solutions of the serum type, solutions of the purified serum type, compounds of the antibody type, compounds of the monoclonal antibody type, fragments of such antibodies, notably Fab, F (ab')2, CDR fragments in so far these fragments have retained the capacity to bind to a polypeptide according to any one of claims 1-5, and humanized antibodies.

**8.** Product according to claim 6, **characterized in that** it does not bind to other known transcription factor(s) or transcription modulator(s).

**9.** Isolated polypeptide such as obtained by isolation from a cell with the help of a product according to any one claims 7-8.

**10.** Product according to any one of claims 7-8, **characterized in that** it is capable of inhibiting or blocking the biological activity of their target polypeptide, such as those binding products of the invention which bind to a nuclearization signal region of a polypeptide according to any one of claims 1-5, 9.

**11.** Product according to any one of claims 7-8, **characterized in that** it is capable of stimulating the biological activity of their target polypeptide, such as those which stabilize the formation of complexes involving a polypeptide according to any one of claims 1-5, 9.

**12.** Isolated complex comprising at least one polypeptide according to any one of claims 1-6, 9.

**13.** Isolated complex which comprises at least one polypeptide according to any one of claim 1-6, 9, together with at least one product chosen among the group consisting of the transcription factors and transcription modulators, such as E2F-4, E2F-5, DP-1, DP-2, pRB, p130, p107, the polynucleotidic molecules such as any DNA molecule, and particularly any nuclear DNA molecule.

**14.** Isolated complex according to claim 13, **characterized in that** said DNA molecule is useful in antioncogeny, such as p53.

**15.** Isolated polynucleotide characteristic of a post-mitotic state, **characterized in that** its sequence codes for a polypeptide comprising:

- at least one sequence of the leucine zipper type at the N-terminus,
- at least one sequence of the basic domain type at the C-terminus, and
- at least one sequence of the nuclearization signal type.

**16.** Isolated polynucleotide chosen among the group consisting of:

- polynucleotides of which sequence corresponds to a sequence coding for SEQ ID N°2, 3, 5 and their human variant homologues,
- polynucleotides of which sequence shows an homology of at least 50%, particularly of at least 60%, notably of at least 70%, preferably of at least 80%, most preferably of at least 90% with SEQ ID N°1, or SEQ ID N°4 while retaining the corresponding biological activity of the encoded product,
- polynucleotides of which sequence corresponds to SEQ ID N°1 or SEQ ID N°4.

**17.** Isolated polynucleotide of which sequence corresponds to a sequence chosen among the group consisting of SEQ ID N°6, SEQ ID N°9, SEQ ID N°19, SEQ ID N°7, SEQ ID N°8, SEQ ID N°10, SEQ ID N°11, SEQ ID N°12, SEQ ID N°13, SEQ ID N°14, SEQ ID N°15, SEQ ID N°21, SEQ ID N°22, SEQ ID N°23.

**18.** Polynucleotidic probe chosen among the group consisting of SEQ ID N°1, SEQ ID N°4, SEQ ID N°6, SEQ ID N°9, SEQ ID N°19, SEQ ID N°7, SEQ ID N°8, SEQ ID N°10, SEQ ID N°11, SEQ ID N°12, SEQ ID N°13, SEQ ID N°14, SEQ ID N°15, SEQ ID N°21, SEQ ID N°22, SEQ ID N°23.

**19.** Couple of primers chosen among the group consisting of SEQ ID N°7 and 8, SEQ ID N°10 and 11, SEQ ID N°12 and 13, SEQ ID N°14 and 15.

**20.** Isolated polynucleotide such as obtained by:

screening a polynucleotidic library, and in particular of cDNA or mRNA library, with at least one probe or one polynucleotide according to any one of claims 15-18, and/or
amplifying a polynucleotidic population with at least one primer couple according to claim 19.

**21.** Isolated polynucleotide which is the antisens of a polynucleotide according to any one of claims 15-18, 20.

**22.** Isolated polynucleotide which is the antisens of a polynucleotide according to any one of claims 15-18, 20, and overlaps the initiation codon of the target polynucleotide.

**23.** Isolated polynucleotide according to claim 21 or 22, **characterized in that** its sequence corresponds to SEQ ID N°24.

**24.** Isolated polypeptide of which sequence is such as obtained by:

i. screening a polynucleotidic library, and in particular of cDNA or mRNA library, with at least one probe or one polynucleotide according to any one of claims 15-18, 20, sequencing the ORF corresponding to the polynucleotide detected by said probe or polynucleotide, or

amplifying a polynucleotidic population with at least one primer couple according to claim 19, sequencing the ORF corresponding to the amplified products, and
ii. deducing according to the universal genetic code the polypeptidic sequence corresponding to the identified ORF.

**25.** Transfection vector which comprises at least one polynucleotide according to any one of claims 15-18, 20, or at least one polynucleotide coding for a complex according to any one of claims 12-14.

**26.** Transfection vector which comprises at least one polynucleotide according to any one of claims 15-18, 20, or at least one polynucleotide coding for a complex according to anyone of claims 12-14, and which further comprises a promotor which allows the targetting of a particular cell type.

**27.** Cell transfected by a polynucleotide according to any one of claims 15-18, 20, by a polynucleotide coding for a complex according to any one of claims 12-14, or by a transfection vector according to any one of claims 25-26.

**28.** Transfection vector which comprises at least one antisens polynucleotide according to any one of claims 21-23.

**29.** Cell transfected by an antisens polynucleotide according to any one of claims 21-23, or by a transfection vector according to claim 28.

**29.** Drug nuclearization vector comprising at least one polynucleotide according to any one of claims 15-17, 20, or at least one polynucleotide coding for a complex according to any one of claims 12-14, these polynucleotides either comprising a sequence coding for a nuclearization signal or being coupled to a nuclearization compound.

**30.** Drug nuclearization vector according to claim 29, **characterized in that** said drug is intended for treatment of the nucleus of a cell, *e.g.* for inhibiting mitosis, such as the p53 antioncogene.

**31.** Pharmaceutical composition, and in particular composition for the therapy and/or palliation and/ or prevention of an undesired state, **characterized in that** it comprises, in a quantity effective for the desired effect, at least one polypeptide according to any one of claims 1-6, 9, 24, and/or at least one product according to claim 11, and/or at least one polynucleotide according to any one of claims 15-17, 20, and/or at least one complex according to any one of claims 12-14, and/or at least one polynucleotide coding for a complex according to any one of claims 12-14, and/or at least one transfection vector coding for a complex according to any one of claims 25-26, and/or at least one transfected cell coding for a complex according to claim 27.

**32.** Pharmaceutical composition, and in particular composition intended for the therapy and/or palliation and/ or prevention of an undesired state, **characterized in that** it comprises, in a quantity effective for the desired effect, at least one product according to claim 10, and/or at least one antisens polynucleotide according to any one of claims 21-23, and/or at least one transfection vector according to claim 28, and/or at least one cell according to claim 29.

**33.** Reagent or kit which comprise at least one product chosen among the group consisting of the polypeptides according to any one of claims 1-6, 9, 24, the products according to any one of claims 7-8, the polynucleotides, probes and primers according to any one of claims 15-23, the complexes according to any one of claims 12-14, the transfection vectors according to any one of claims 25-26, 28, the cells according to any one of claims 27 or 29.

**34.** A method for diagnosing an undesired state of a human person or of an animal, or for following the evolution of this undesired state, namely along the course of a medical treatment, comprising:

- the observation in a biological sample of polypeptides according to any one of claims 1-6, 9, 24 at a level significantly inferior to the normal level in the nuclear part of said biological sample, and/or
- the observation in a biological sample of polypeptides according to any one of claims 1-6, 9, 24 at a level significantly higher in the cytoplasmic part of this biological sample compared to the level observed in the nuclear part of the same sample.

**35.** The method of claim 34, **characterized in that** said observation is made with the use of at least one product according to any one of claims 7 or 8.

**36.** A method according to claim 34 or 35, **characterized in that** said undesired state corresponds to a cancerous state.

**37.** A method for identifying an anti-mitotic agent, **characterized in that**:

- a test compound is placed in contact with a cell which expresses at least one polypeptide according to any one of claims 1-6, 9, 24 in a compartment different from the nucleus,
- said test compound is identified as an anti-mitotic agent if it can be observed that said at least one polypeptide has moved towards or into the nucleus.

# FIGURE 1A

cagcagcagtacttgaatggcttagggcagagagtctgaagagagtcgagttgcacagtgaaaatatatatt 72
tttatacctagcacaacttccttgtatatttttggggttcttttattgtttgtttgtttttttttcctttttat 144
tttttctttttcctttcctttttttgttttggttcttcttcgatctgggttcgttttattacttcttcttatt 216
actaggaaggcaaacttctaaaatgtgaagggcgttgggtttctccttatctggtagatgagggcaagcatt 288
ttgcactaatgtttcctgtgtgtggaggattcatggtatgtttggaacatgttacgtggataatgattgtat 360
tcttccgacaaacacaaccaagttccccctttggaaatactgaatgtaaatgagaacagagcagtccatcga 432
tttgtatgcggctttcctgagtgccttagaatctcttgatctcttttgttccttcttttgttgtcggtttgg 504
tttggtttgggtttttgttgggttttctttttttcttttccaaggaaaacagatggagagaatcagctcataa 576
ccacctccttcctcctctttgtttacgatactattttaaattatagaattcggggctgctttataatgtgaa 648
taggaaactgcgttcatctaggtctgatagtgtaaaatcagagcatcagtatttatggtatgtctgcacctt 720

M Y I I Y L K L Y Q -742
tattcctatgcttgactattagcaatattac ATG TAT ATT ATA TAT CTA AAG TTA TAC CAA 781

A P L K K S E R V L R A V P T S L H -724
GCA CCT TTA AAG AAA TCA GAG CGC GTG CTC CGT GCA GTC CCC ACA TCC CTA CAT 835

L H I H A S L Y P D I P T S P H P Y -706
CTC CAT ATC CAT GCA TCC CTA TAT CCT GAC ATC CCT ACA TCA CCG CAT CCC TAT 889

I T V S P H P C I L I S P H I S L S -688
ATC ACT GTA TCT CCA CAT CCC TGT ATC CTC ATA TCC CCA CAT ATC TCC TTA TCC 943

P H P H F P A P S S C R T L L L P A -670
CCA CAC CCC CAT TTC CCA GCT CCC AGC AGC TGT AGG ACC CTC CTC CTC CCA GCT 997

V S R Q Q R V P S P S H Y G V H M D -652
GTG TCC CGG CAG CAG CGG GTG CCG TCC CCA TCC CAC TAT GGG GTG CAC ATG GAC 1051

C G L M E P P S R E S I A F P D R F -634
TGT GGC CTC ATG GAG CCA CCG AGC AGG GAA TCG ATT GCC TTT CCT GAT CGT TTT 1105

P A K L M L G L L I F T E I T C C E -616
CCT GCT AAG CTT ATG TTG GGA TTG CTT ATA TTT ACA GAA ATA ACT TGC TGT GAG 1159

R R R V Y G P L T N P K P Q S L S A -598
CGG AGA AGG GTG TAT GGC CCA TTA ACC AAC CCA AAA CCC CAG AGC CTC TCG GCT 1213

I N P K G G T E N S R L F W G S N C -580
ATA AAT CCT AAA GGC GGC ACA GAG AAC TCA CGT CTC TTC TGG GGC TCC AAC TGC 1267

C L E V F L S Y R C V T L S A Q G R -562
TGT TTA GAA GTG TTC CTG TCG TAT AGA TGT GTC ACG TTG TCA GCC CAG GGT CGG 1321

G T P K P G L S G A P C T D R R A M -544
GGC ACC CCA AAG CCA GGG CTG TCA GGA GCT CCG TGC ACC GAT AGG CGT GCC ATG 1375

S F F H C Y R S I L H T K V G F Y V -526
AGT TTC TTC CAC TGT TAT AGA AGC ATT TTG CAC ACC AAG GTT GGT TTT TAT GTC 1429

K I K K K K R K K K R N N N N N N K -508
AAA ATT AAG AAG AAA AAA AGA AAA AAA AAA AGA AAT AAT AAT AAT AAT AAT AAA 1483

N N N N K Y I Y I L K R K E N K A K -490
AAT AAT AAT AAT AAA TAT ATA TAT ATA TTA AAA AGA AAA GAA AAC AAA GCC AAA 1537

T C N T V F S R R N K S T N K * -474
ACT TGC AAT ACT GTT TTT AGC AGA CGG AAT AAA TCC ACG AAC AAA TAA cgagatg 1592
aaaggaatgaatgagtgggtggaaaacgacgcctgtagggaaaggctctgaactgtcctttggggcagggggg 1664
cgttgggttgggtcagagtgctgaccaacttgggctgcttcaggggaaggcaaaatgaagggttgagtggtg 1736
ccgttgttttggaggcggctccgcgctgtgcgcagtactgtgactccacggactgttatccctgtgtgtgct 1808
gtaggacagccccgcagtacgctgagctccggctctcagccacggacgatgctcgcttcgtcttggttcttt 1880
ttgattttgactttgatcgcgcattattcaccggtatttatgtgctctttactctgtgtagttctgactgtt 1952
attttgtagccacgctgatgccatatagaggtaactaaaaccagaaataaaaggttaaggacaaaccccatc 2024
ccccccctccctttaccccgctaccctctatcggatctgtgctcacagctgttttcagacgacggaaaatcca 2096
tttaagaggaacgaatttcgcggccggtgcgaccatgaattgtacaacgctttgtaaagttcacatttacag 2168
aataataaaggaaatgatgattacaaac 2196

FIGURE 1B

Leucine Zipper Domain

**MYIIYLKLYQAPLKKSERVLRAVPTSLHLHIHASLYPDIPTSPHPYITVSPHPCILISPHISLSPHPHFPA 71**

**PSSCRTLLLPAVSRQQRVPSPSHYGVHMDCGLMEPPSRESIAFPDRFPAKLMLGLLIFTEITCCERRRVYGP 143**

**LTNPKPQSLSAINPKGGTENSRLFWGSNCCLEVFLSYRCVTLSAQGRGTPKPGLSGAPCTDRRAMSFFHCYR 215**

**SILHTKVGFYVKIKKKKRKKKRNNNNNNKNNNNKYIYILKRKENKAKTCNTVFSRRNKSTNK 277**

Nuclear Localisation Signal

Basic Domain

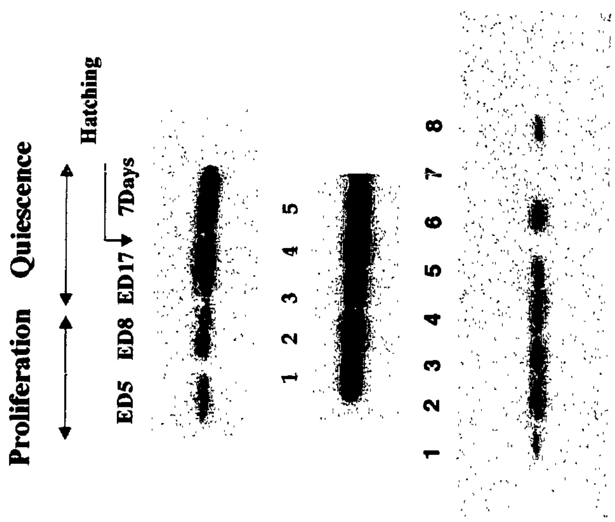


Figure 2A

Figure 2B

Figure 2C

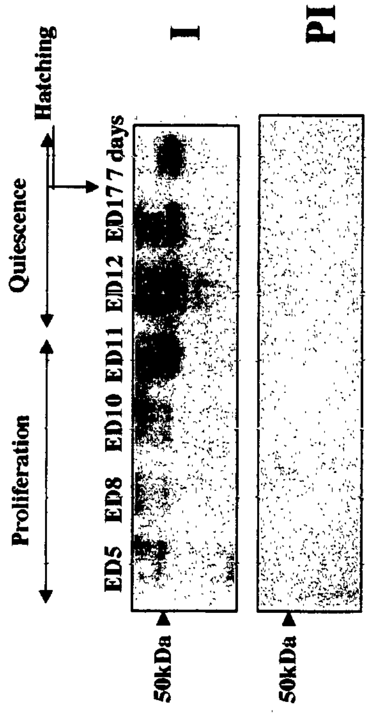


Figure 3A

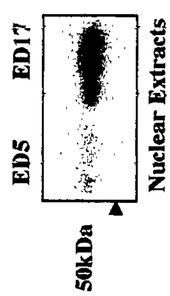


Figure 3B

FIGURE 4A

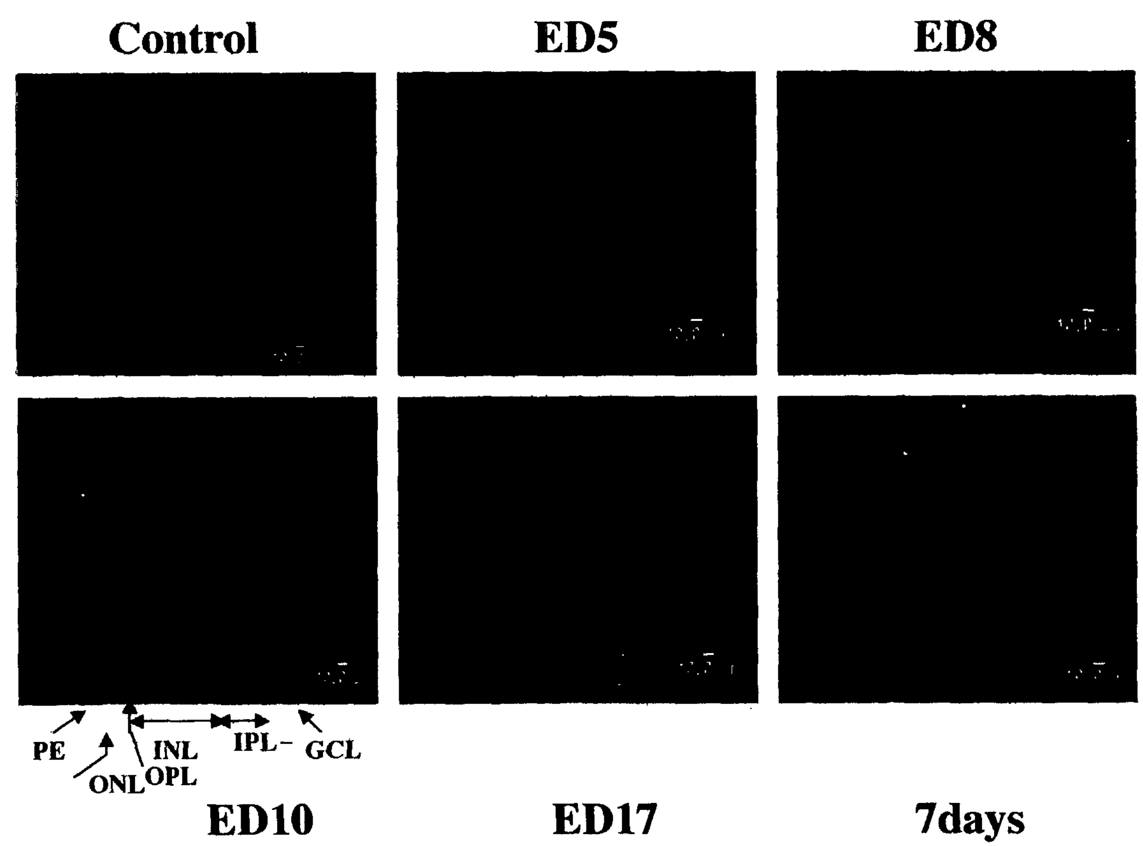

FIGURE 4B

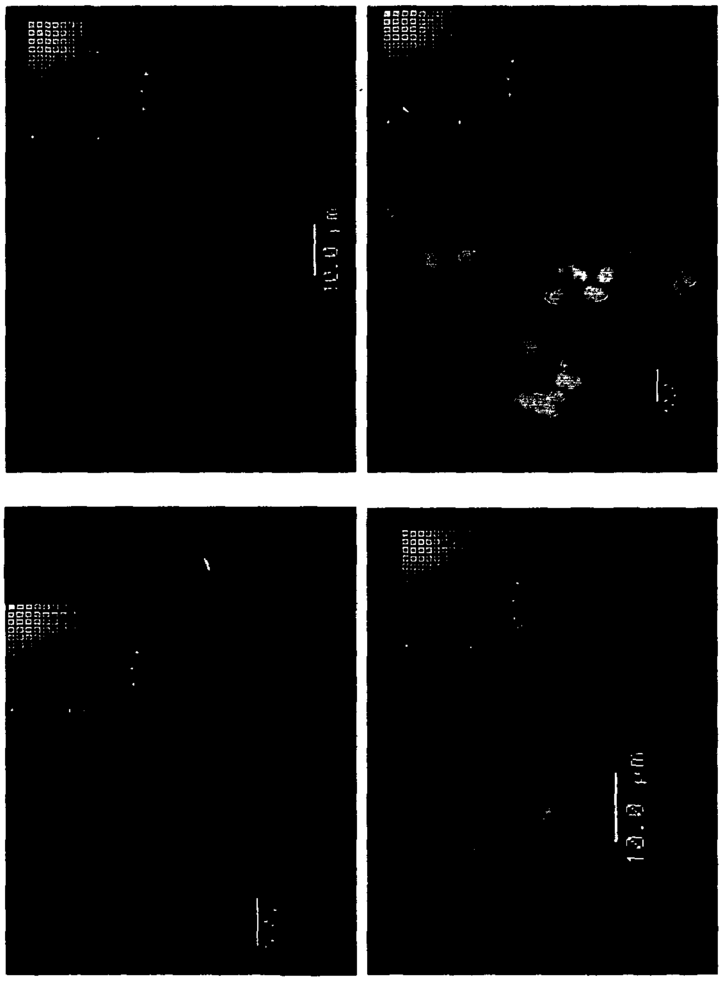

Control

ED5

ED8

ED17

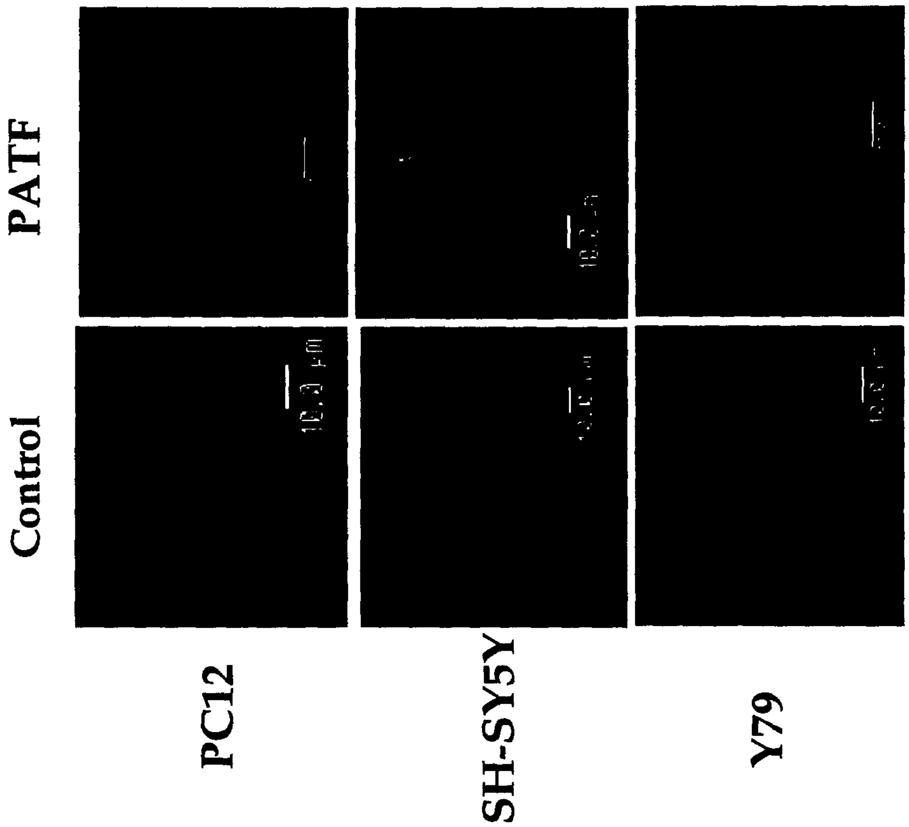


FIGURE 5

FIGURE 6A

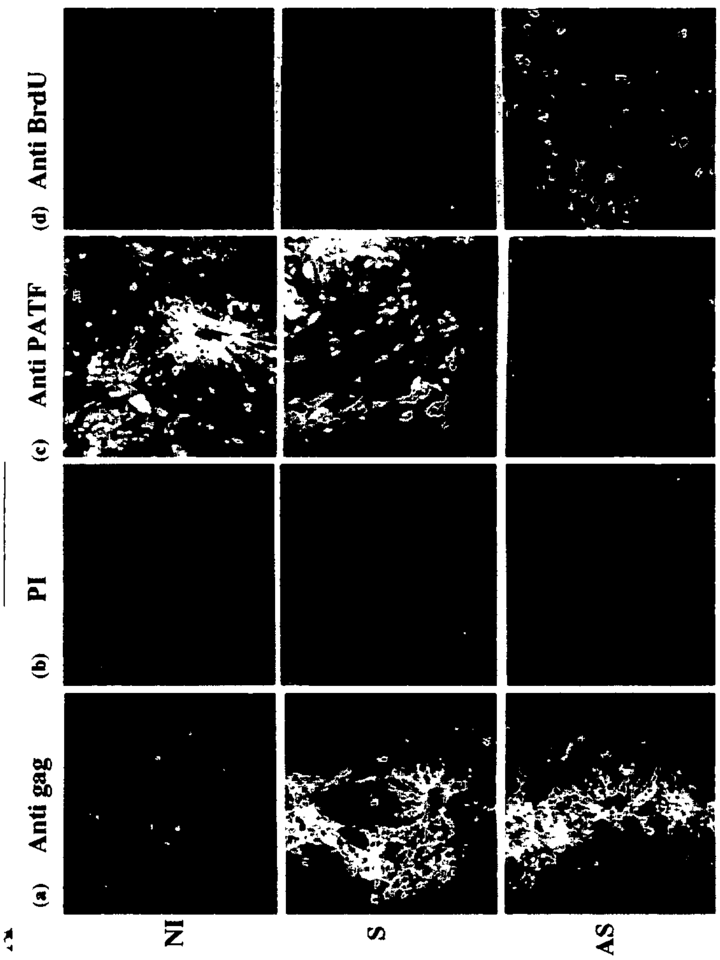

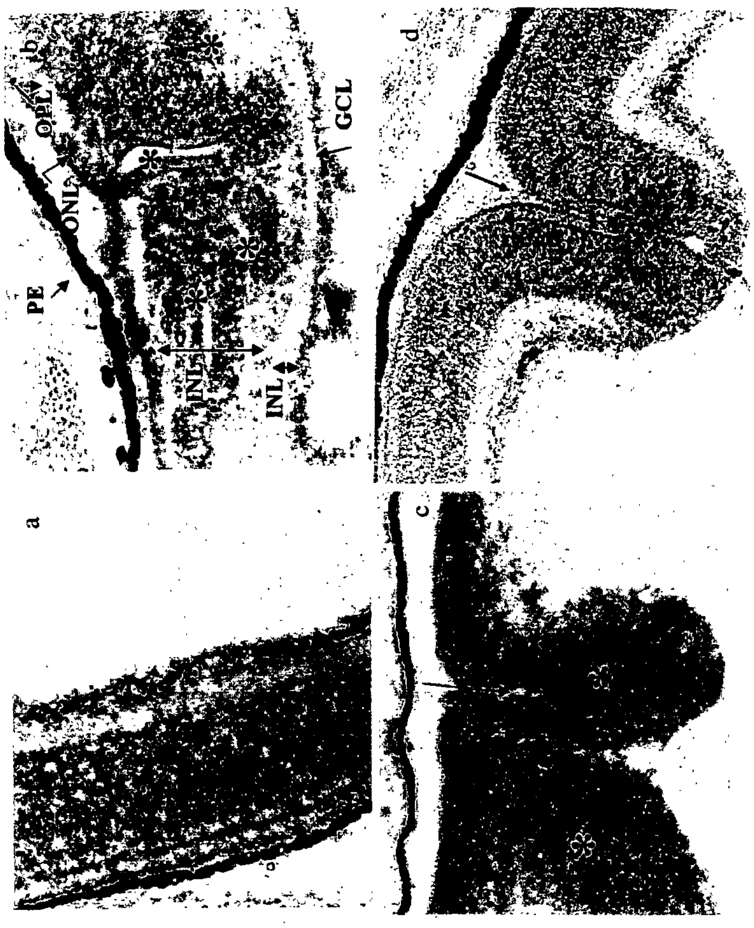


FIGURE 6B

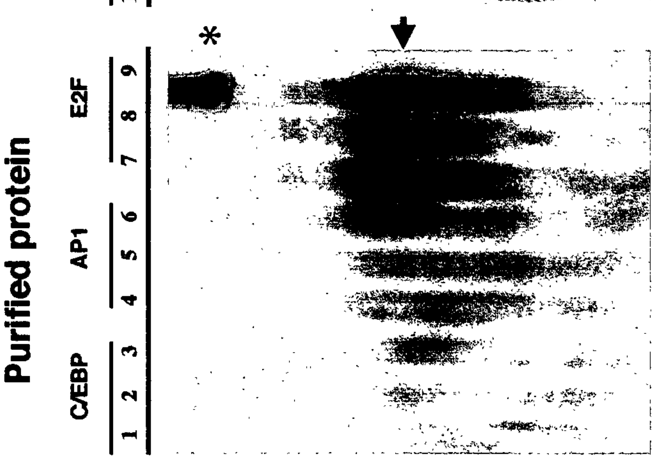


FIGURE 7A

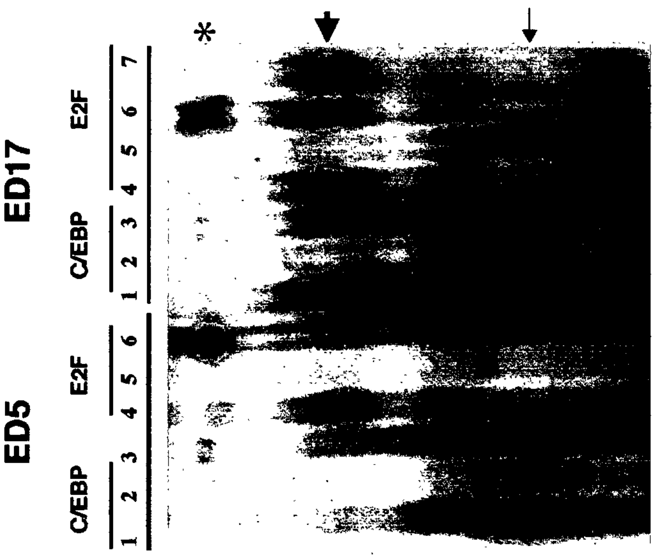


FIGURE 7 B

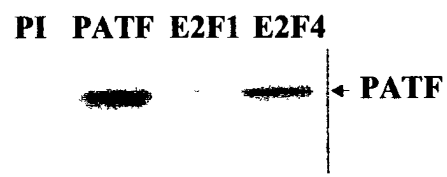


FIGURE 8A

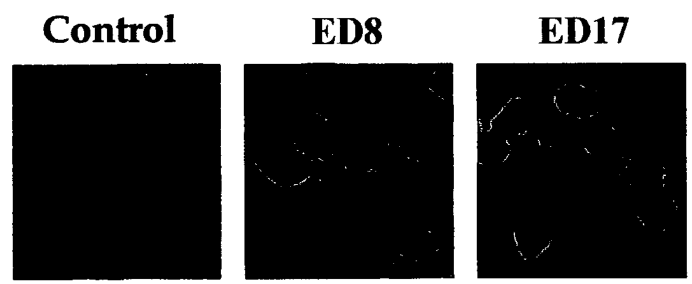


FIGURE 8B

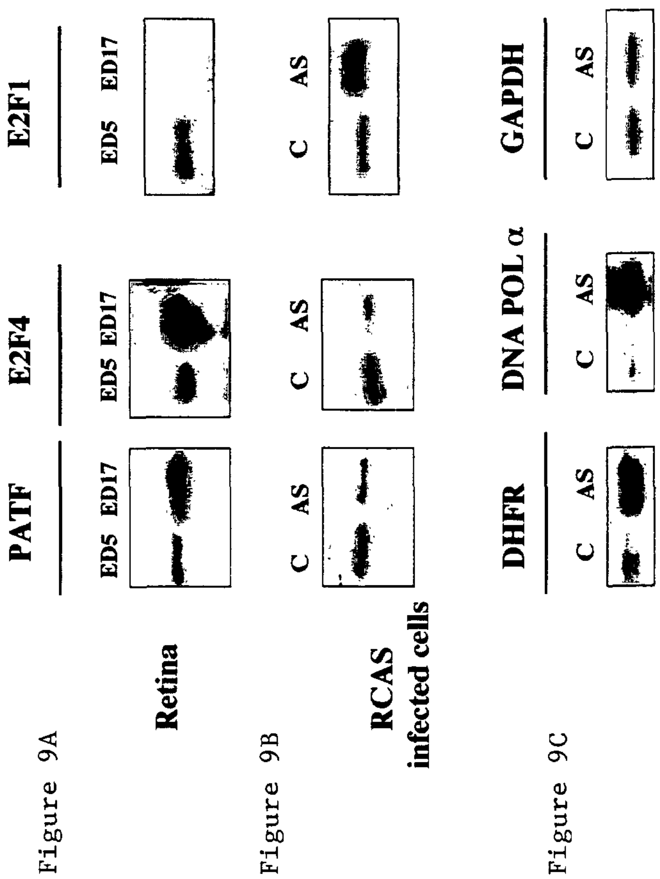


Figure 9A

Figure 9B

Figure 9C

European Patent Office

# PARTIAL EUROPEAN SEARCH REPORT

which under Rule 45 of the European Patent Convention shall be considered, for the purposes of subsequent proceedings, as the European search report

Application Number EP 00 40 0588

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X,D | BIDOU L ET AL: "A novel cDNA corresponding to transcripts expressed in retina post-mitotic neurons" MECHANISMS OF DEVELOPMENT, vol. 43, 1993, pages 159-173, XP000925814 SHANNON IR * see thw whole dicouent, especially page 162, column 1 * --- | 15,16, 18,20 | C12N15/12 C07K14/47 C07K16/18 C12Q1/68 C12N15/11 C12N5/10 C12N15/85 A61K38/17 A61P35/00 |
| X | BJERKNES M ET AL: "TCFL4: a gene at 17q21.1 encoding a putative basic helix-loop-helix leucine-zipper transcription factor" GENE: AN INTERNATIONAL JOURNAL ON GENES AND GENOMES,GB,ELSEVIER SCIENCE PUBLISHERS, BARKING, vol. 181, no. 1-2, 28 November 1996 (1996-11-28), pages 7-11, XP004071851 ISSN: 0378-1119 * the whole document * --- | 1-3 | |
| | -/-- | | TECHNICAL FIELDS SEARCHED (Int.Cl.7) C07K |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| BERLIN | 10 August 2000 | De Kok, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**INCOMPLETE SEARCH SHEET C**

Application Number
EP 00 40 0588

Claim(s) searched completely:
4-6, 16-19, 23

Claim(s) searched incompletely:
1-3, 7-15, 20-22, 24-37

Reason for the limitation of the search:

Present independent claims 1 and 15 relate to an extremely large number of possible polypeptides and corresponding polynucleotides. Support within the meaning of Article 84 EPC and/or disclosure within the meaning of Article 83 EPC is to be found, however, for only one of the polypeptides/polynucleotides claimed, namely the proliferation arrest transcription factor (PATF) polypeptide and its corresponding DNA. In the present case, the claims so lack support, and the application so lacks disclosure, that a meaningful search over the whole of the claimed scope is impossible. Consequently, the search has been carried out for those parts of the claims which appear to be supported and disclosed, namely those parts relating to PATF polypeptides and nucleic acids encoding these peptides and theire uses, i.e. the subject-matter of claims 4-6, 16-19 and 23 as well as claims 1-3, 7, 12-15, 20-22, 24-37 as far as there subject-matter refers to PATF polypeptides, c.q. nucleic acids.

Present claims 7-11 relate to a product defined by reference to a desirable property, namely capable of binding to a polypeptide as defined in claims 1-6, as well as products derived from or isolated by use of those products.
The claims cover all products having this characteristic or property, whereas the application provides support within the meaning of Article 84 EPC and/or disclosure within the meaning of Article 83 EPC for only one such products. In the present case, the claims so lack support, and the application so lacks disclosure, that a meaningful search over the whole of the claimed scope is impossible. Independent of the above reasoning, the claims also lack clarity (Article 84 EPC). An attempt is made to define the product by reference to a result to be achieved. Again, this lack of clarity in the present case is such as to render a meaningful search over the whole of the claimed scope impossible. Consequently, the search has been carried out for those parts of the claims which appear to be clear, supported and disclosed, namely those parts relating to the monoclonal antibodies reacting with PATF or fragments thereof.

European Patent Office

# PARTIAL EUROPEAN SEARCH REPORT

Application Number
EP 00 40 0588

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WILLIAMS S ET AL.: "C/EBP proteins contain nuclear localisation signals embedded in their basic regions" GENE EXPRESSION, vol. 6, 1997, pages 371-385, XP000925836 US<br>* page 371 - page 372 * | 1-3 | |
| X | GECK P ET AL.: "Early gene expression during androgen-induced inhibition of proliferation of prostate cancer cells: A new suppressor candidate on chromosome 13, in the BRCA2-Rb1 locus." JOURNAL OF STEROID BIOCHEMISTRY AND MOLECULAR BIOLOGY, vol. 68, 1999, pages 41-50, XP000925835 GB<br>* abstract *<br>* page 41 - page 42 *<br>* page 46 - page 47 * | 1-3 | TECHNICAL FIELDS SEARCHED (Int.Cl.7) |
| X | DE 198 15 331 A (KARLSRUHE FORSCHZENT) 14 October 1999 (1999-10-14)<br>* the whole document * | 1 | |

EPO FORM 1503 03.82 (P04C10)

European Patent Office

Application Number

EP 00 40 0588

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-16 completely, 17-22 partly, 23 completely, 24-33 partly and 34-37 completely

European Patent Office

## LACK OF UNITY OF INVENTION SHEET B

Application Number
EP 00 40 0588

The Search Division considers that the present European patent application does not comply with the requirements of unity of invention and relates to several inventions or groups of inventions, namely:

1. Claims: 1-16 completely, 17-22 partly, 23 completely, 24-33 partly and 34-37 completely

   An isolated polypeptide with a sequence corresponding to SEQ.ID.No. 2, 3 or 5; peptides derived therefrom having SEQ.ID.No.21, 22 or 23; monoclonal antibodies agains said polypeptides; isolated complex comprising said polypeptides; isolated polynucleotides encoding said polypeptides, especially SEQ.ID.No.1 and 4; probes, primers, antisens molecules derived from said polynucleotides; a transformation vector, host cell and nuclearization vector comprising said polynucleotides; pharmaceutical compositions or kits comprising said polypeptides c.q. polynucleotides; a method for diagnosing disease state and a method for identifying an antimitotic agent.

2. Claims: 17-22, 24-33 all partly

   An isolated polynucleotide related to the DHFR gene with a sequence corresponding to SEQ.ID.No. 10 or 11; probes, primers, antisens molecules derived from said polynucleotides; a transformation vector, host cell and nuclearization vector comprising said polynucleotides; pharmaceutical compositions or kits comprising said polynucleotides, as well as products obtained by the use of said polynucleotides.

3. Claims: 17-22, 24-33 all partly

   An isolated polynucleotide related to the DNA polymerase alpha gene with a sequence corresponding to SEQ.ID.No. 12 or 13; probes, primers, antisens molecules derived from said polynucleotides; a transformation vector, host cell and nuclearization vector comprising said polynucleotides; pharmaceutical compositions or kits comprising said polynucleotides, as well as products obtained by the use of said polynucleotides.

4. Claims: 17-22, 24-33 all partly

   An isolated polynucleotide related to the GAPDH gene with a sequence corresponding to SEQ.ID.No. 14 or 15; probes, primers, antisens molecules derived from said polynucleotides; a transformation vector, host cell and nuclearization vector comprising said polynucleotides; pharmaceutical compositions or kits comprising said polynucleotides, as well as products obtained by the use of said polynucleotides.

European Patent Office

# LACK OF UNITY OF INVENTION SHEET B

Application Number
EP 00 40 0588

The Search Division considers that the present European patent application does not comply with the requirements of unity of invention and relates to several inventions or groups of inventions, namely:

## ANNEX TO THE EUROPEAN SEARCH REPORT ON EUROPEAN PATENT APPLICATION NO. EP 00 40 0588

This annex lists the patent family members relating to the patent documents cited in the above-mentioned European search report.
The members are as contained in the European Patent Office EDP file on
The European Patent Office is in no way liable for these particulars which are merely given for the purpose of information.

10-08-2000

| Patent document cited in search report | Publication date | Patent family member(s) | Publication date |
|---|---|---|---|
| DE 19815331 A | 14-10-1999 | AU 2934399 A | 25-10-1999 |
| | | WO 9951635 A | 14-10-1999 |

EPO FORM P0459

For more details about this annex : see Official Journal of the European Patent Office, No. 12/82